# EUROPEAN PATENT APPLICATION

(11) **EP 4 257 678 A1**
(43) Date of publication of application: **11.10.2023**
(21) Application number: 21903397.4
(22) Date of filing: 07.12.2021
(51) Int. Cl.: C12N 5/10, C12N 15/09

(54) **PRODUCTION METHOD FOR PRODUCING PLURIPOTENT STEM CELL POPULATION**

(30) Priority: 07.12.2020 JP 2020202566
(71) Applicant: Kaneka Corporation, Osaka-shi, Osaka 530-8288 (JP); RIKEN, Wako-shi, Saitama 351-0198 (JP)
(72) Inventor: KAMBAYASHI, Sho, Kobe-shi, Hyogo 650-0047 (JP); HAYASHI, Yohei, Wako-shi, Saitama 351-0198 (JP); TAKASAKI, Mami, Wako-shi, Saitama 351-0198 (JP)
(74) Representative: Hoffmann Eitle
(86) International application number: PCT/JP2021/044881
(87) International publication number: WO 2022/124298

(57) **Abstract**

This invention is intended to suppress cell death occurring at the time of transition of adherent culture of pluripotent stem cells to suspension culture thereof. Pluripotent stem cells are subjected to adherent culture in a liquid medium comprising a PKCβ inhibitor and a TNKS inhibitor and then to suspension culture.

## Description

### Technical Field

The present invention relates to a method for producing a pluripotent stem cell population through a procedure of adherent culture to suspension culture of pluripotent stem cells.

### Background Art

Pluripotent stem cells, such as ES cells and iPS cells, can grow indefinitely and can differentiate into a wide variety of somatic cells. If a therapeutic method comprising transplanting somatic cells induced to differentiate from pluripotent stem cells is realized, therapeutic methods for intractable diseases and lifestyle-related diseases can be radically changed. For example, techniques of inducing pluripotent stem cells to differentiate into a wide variety of somatic cells, such as nerve cells, cardiac muscle cells, blood cells, and retinal cells, *in vitro* have already been developed.

Meanwhile, regenerative medicine using pluripotent stem cells has problems to solve toward the practical use thereof, and one of such problems is productivity of pluripotent stem cells. For example, it is said that approximately 2 × 10¹¹ cells are necessary to reproduce a liver. Methods of pluripotent stem cell culture are roughly classified into adherent culture that is performed by allowing cells to adhere to a flat culture substrate (plate) and suspension culture that is performed by allowing cells to be suspended in a liquid medium. In order to culture cells of the number as mentioned above via adherent culture, it is necessary to use a culture substrate (plate) with a size of 10⁶ cm² or larger. When a common-typical 10-cm-dish is to be used, it is necessary to prepare approximately 20,000 dishes. In the case of adherent culture performed on a culture substrate (plate) surface, as described above, the number of cells obtained thereby depends on an area of culture. Accordingly, an enormous area is required to increase the culture scale, and it is difficult to supply the amount of cells necessary for regenerative medicine. In contrast, suspension culture is performed while allowing cells to be suspended in a liquid medium, and the number of cells obtained thereby thus depends on a medium volume. Accordingly, it may be relatively practical to increase the scale of suspension culture, and suspension culture may be suitable for mass production of cells. For example, Non-Patent Document 1 discloses a method of suspension culture of pluripotent stem cells, which is performed with the use of a spinner flask as a cell culture vessel for suspension culture while stirring a liquid medium. Patent Document 1 discloses a method of efficiently forming a cell aggregate for suspension culture by dispensing pluripotent stem cells dispersed in a single cell into a vessel in which the center of the well bottom has a concave curved surface.

In order to achieve the effects of interest, such as an improved growth efficiency or maintenance of the undifferentiated state, it is known that various substances are added to a medium used for suspension culture or adherent culture of pluripotent stem cells. Patent Document 2 discloses that pluripotent stem cells are subjected to suspension culture in the presence of a Wnt signal inhibitor. Patent Document 3 discloses a medium for stem cell culture, which contains a PKC (protein kinase C) inhibitor and so on, and contains no or a small amount of growth factor. Patent Document 4 discloses a method for human pluripotent stem cell culture in which culture is performed with the addition of a PKC inhibitor to a medium to maintain the undifferentiated state of cells with a high probability. Patent Document 5 discloses a method of culture while maintaining the undifferentiated state of primed pluripotent stem cells, in which pluripotent stem cells are cultured in a medium containing a histone deacetylase inhibitor and so on, and then culture in a medium not containing the histone deacetylase inhibitor but containing the MAPK/ERK kinase inhibitor, a PKC inhibitor, a Wnt signaling inhibitor, and the leukemia inhibitory factor.

### Prior Art Documents

### Non-Patent Documents

Non-Patent Document 1: Olmer R.et al., Tissue Engineering: Part C, Volume 18 (10): 772-784, 2012

### Patent Documents

Patent Document 1: WO 2013/183777 A1
Patent Document 2: WO 2020/039732 A1
Patent Document 3: WO 2018/047941 A1
Patent Document 4: JP 2012-175962 A
Patent Document 5: WO 2019/151386 A1

### Summary of the Invention

### Objects to Be Attained by the Invention

Culture of pluripotent stem cells is often performed via adherent condition, and suspension culture method remains relatively unknown. Accordingly, the efficiency of transition from adherent culture to suspension culture has not been sufficiently examined, and such efficiency remains low. In order to perform suspension culture of pluripotent stem cells, specifically, it is necessary to transfer adherent culture to suspension culture. When the cells are transferred to suspension culture, disadvantageously, many cells would die. A greater extent of the death of pluripotent stem cells at the time of transition from adherent culture to suspension culture would necessitate a longer period of time to recover the original number of cells and perform expansion culture.

An object of the present invention is to provide a method for preparing pluripotent stem cells that enables efficient culture of pluripotent stem cells by suppressing the cell death occurring at the time of transition from adherent culture to suspension culture.

### Means for Attaining the Object

The present inventors have conducted intensive studies in order to attain the above object. As a result, the present inventors discovered that pluripotent stem cells could be prevented from cell death after the transition from adherent culture to suspension culture by performing the adherent culture in the presence of a PKCβ inhibitor and a TNKS inhibitor in a method for producing a pluripotent stem cell population comprising subjecting pluripotent stem cells to a procedure of adherent culture followed by suspension culture. This has led to the completion of the present invention.

The present invention includes the following.

(1) A method for producing a pluripotent stem cell population comprising: a step of adherent culture of pluripotent stem cells in a liquid medium comprising a PKCβ inhibitor and a TNKS inhibitor; and a step of suspension culture of the pluripotent stem cells after the adherent culture.
(2) The method according to (1), wherein concentration of the PKCβ inhibitor in the liquid medium is 25 nM to 15 µM.
(3) The method according to (1), wherein concentration of the TNKS inhibitor in the liquid medium is 90 nM to 40 µM.
(4) The method according to (1), wherein a ratio of the content of the PKCβ inhibitor to the content of the TNKS inhibitor in the liquid medium is 167:1 to 1:1600.
(5) The method according to (1), wherein the liquid medium comprises at least one substance selected from the group consisting of L-ascorbic acid, insulin, transferrin, selenium, and sodium bicarbonate.
(6) The method according to (1), wherein the liquid medium comprises FGF2 and/or TGF-β1.
(7) The method according to (1), wherein the liquid medium comprises a ROCK inhibitor.
(8) The method according to (7), wherein the ROCK inhibitor is Y-27632.
(9) The method according to (1), wherein the step of suspension culture comprises a step of forming a cell aggregate.
(10) The method according to (1), wherein the step of suspension culture comprises a step of collecting a cell aggregate.
(11) The method according to (1), wherein, in the pluripotent stem cell population, a proportion of cells positive for OCT4 is 90% or higher, that of cells positive for SOX2 is 90% or higher, and that of cells positive for NANOG is 90% or higher.
(12) The method according to (1), wherein the pluripotent stem cells are ES cells and/or induced pluripotent stem cells.
(13) A pluripotent stem cell population produced by the method according to any of (1) to (12).
(14) An inhibitor of the death of pluripotent stem cells used for adherent culture comprising a PKCβ inhibitor and a TNKS inhibitor.
(15) The inhibitor of the death of pluripotent stem cells used for adherent culture according to (14), wherein the content of the PKCβ inhibitor is 50 nM to 200 mM.
(16) The inhibitor of the death of pluripotent stem cells used for adherent culture according to (14), wherein the content of the TNKS inhibitor is 180 nM to 113 mM.
(17) The inhibitor of the death of pluripotent stem cells used for adherent culture according to (14), wherein a ratio of the content of the PKCβ inhibitor to the content of the TNKS inhibitor is 167:1 to 1:1600.
(18) A kit of a composition for adherent culture of pluripotent stem cells comprising the inhibitor of the death of pluripotent stem cells used for adherent culture according to any of (14) to (17).
(19) The method according to (1) and the inhibitor of the death of pluripotent stem cells used for adherent culture according to (14), wherein the PKCβ inhibitor is at least one member selected from the group consisting of Go6983, GF109203X, LY-333531, Enzastaurin, Sotrastaurin, Ro-31-8220-mesylate, Ro-32-0432-hydrochloride, Go6976, Rottlerin, Midostaurin, Daphnetin, Dequalinium Chloride, Baicalein, Quercetin, Luteolin, Bisindolylmaleimide II, Calphostin C, Chelerythrine chloride, L-threo-Dihydrosphingosine, and Melittin.
(20) The method according to (1) and the inhibitor of the death of pluripotent stem cells used for adherent culture according to (14), wherein the TNKS inhibitor is at least one member selected from the group consisting of IWR-1-endo, XAV939, G007-LK, G244-LM, and WIKI4.
(21) The method according to (1) and the inhibitor of the death of pluripotent stem cells used for adherent culture according to (14), wherein the pluripotent stem cells are primed pluripotent stem cells.
(22) The method according to (1), wherein the liquid medium does not comprise LIF.
(23) The kit of a composition for adherent culture of pluripotent stem cells according to (18), which comprises a LIF-free liquid medium composition.
(24) The method according to (1), wherein the liquid medium does not comprise a GSK3 inhibitor.
(25) The kit of a composition for adherent culture of pluripotent stem cells according to (18), which contains a liquid medium composition not comprising a GSK3 inhibitor.
(26) The method according to (1), wherein the liquid medium does not comprise the GSK3 inhibitor and the MEK/ERK inhibitor.
(27) The kit of a composition for adherent culture of pluripotent stem cells according to (18), which comprises a liquid medium composition not comprising GSK3 inhibitor and MEK/ERK inhibitor.
(28) The method according to (1) and the inhibitor of the death of pluripotent stem cells used for adherent culture according to (14), wherein the PKCβ inhibitor is a compound having the following structural formula (Formula [1]) or a salt thereof.

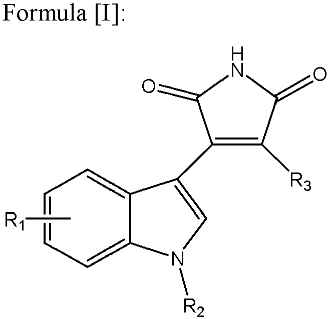

In Formula [I],
R₁ is a hydrogen atom or an alkoxy group having 1 to 3 carbon atoms (preferably a methoxy group);
R₂ is a hydrogen atom, an alkyl group having 1 to 3 carbon atoms (preferably a methyl group), or an alkyl group having 1 to 3 carbon atoms substituted with -N(R_{A})₂ (preferably -(CH₂)₃-N(CH₃)₂);
R_{A} is independently an ethyl group or a methyl group (preferably a methyl group);
R₃ is a group represented by: 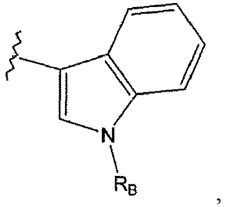 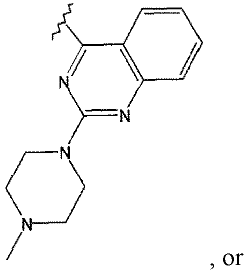 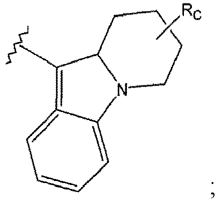
R_{B} is a hydrogen atom, an alkyl group having 2 to 4 carbon atoms substituted with -S-C(=NH)(-NH₂) (preferably -(CH₂)₃-S-C(=NH)(-NH₂)), or a group represented by: 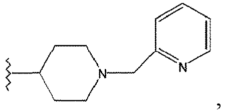
R₂ and R_{B} may together form a divalent group represented by: 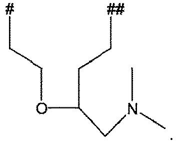 wherein
   a symbol "#" indicates binding of R₂ to a site of binding and a symbol "##" indicates binding of R_{B} to a site of binding,
   a steric configuration of asymmetrical carbon included in the divalent group is preferably represented by, but is not particularly limited to: 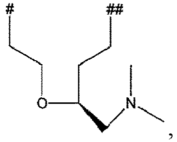
   Rc is an alkyl group having 1 to 3 carbon atoms substituted with -N(R_{D})₂ (preferably -(CH₂)-N(CH₃)₂), and
   R_{D} is independently an ethyl or methyl group (preferably a methyl group).

Examples of a salt of a compound represented by Formula I include a hydrochloride and a sulfate.

(29) The method according to (1), wherein, in the step of suspension culture of pluripotent stem cells, the pluripotent stem cells after the adherent culture are seeded in a liquid medium not comprising PKCβ inhibitor and/or TNKS inhibitor.

### Effects of the Invention

According to the present invention, when pluripotent stem cells are subjected to transition from adherent culture to suspension culture, pluripotent stem cells can be prevented from death, and a pluripotent stem cell population can be produced efficiently.

### Brief Description of the Drawings

Fig. 1 shows characteristic diagrams demonstrating the expression levels of OCT4 gene, NANOG gene, and SOX2 gene when pluripotent stem cells are subjected to adherent culture by the methods described in Example 1 and Comparative Example 1.
Fig. 2 shows characteristic diagrams demonstrating the results of flow cytometry analysis of samples treated with anti-OCT4, anti-SOX2, and anti-NANOG antibodies when pluripotent stem cells are subjected to adherent culture by the methods described in Example 1 and/or Comparative Example 1.

### Embodiments of the Invention

### 1. A method for producing a pluripotent stem cell population

### 1-1. Summary

According to the method for producing a pluripotent stem cell population in the present invention, pluripotent stem cells are first subjected to adherent culture in a liquid medium containing a protein kinase Cβ (PKCβ) inhibitor and a tankyrase (TNKS) inhibitor and then to suspension culture, so as to produce a pluripotent stem cell population. According to the method for producing a pluripotent stem cell population according to the present invention, death of pluripotent stem cells occurring at the time of transition from adherent culture to suspension culture can be prevented, and a pluripotent stem cell population can be produced efficiently.

### 1-2. Definition of Terms

The following terms to be used in the present specification will be defined.

### <<Cells>>

A "pluripotent stem cell" as a subject matter of the invention in the present specification refers to a cell having pluripotent capacity (pluripotency) to differentiate into all types of cells constituting a living body and being capable of permanently continuing proliferation with the pluripotency maintained in *in vitro* culture under adequate conditions. More specifically, pluripotency means an ability to differentiate into germ layers constituting an individual (for vertebrates, three germ layers: ectoderm, mesoderm, and endoderm). Examples of such cells include embryonic stem cells (ES cells), embryonic germ cells (EG cells), germline stem cells (GS cells), and induced pluripotent stem cells (iPS cells). An "ES cell" is a pluripotent stem cell prepared from an early embryo. An "EG cell" is a pluripotent stem cell prepared from a fetal primordial germ cell (Shamblott M. J. et al., 1998, Proc. Natl. Acad. Sci., U.S.A., 95: 13726-13731). A "GS cell" is a pluripotent stem cell prepared from a testicular cell (Conrad S., 2008, Nature, 456: 344-349). An "iPS cell" refers to a pluripotent stem cell that has been reprogrammed by introducing a few genes encoding initialization factors into a differentiated somatic cell to bring the somatic cell into an undifferentiated state.

The pluripotent stem cells in the present specification can be cells derived from any multicellular organism. The pluripotent stem cells are preferably cells derived from an animal, and more preferably cells derived from a mammal. Examples of the mammal include a rodent such as a mouse, a rat, a hamster, and a guinea pig, a domestic or pet animal such as a dog, a cat, a rabbit, a bovine, a horse, sheep, and a goat, and a primate such as a human, a rhesus monkey, a gorilla, and a chimpanzee. Cells derived from a human are particularly preferable.

The pluripotent stem cells to be used in the present specification include naive pluripotent stem cells and primed pluripotent stem cells. By definition, a naive pluripotent stem cell is in a state with pluripotency close to that found in the preimplantation inner cell mass, and a primed pluripotent stem cell is in a state with pluripotency close to that found in the postimplantation epiblast. As compared with naive pluripotent stem cells, primed pluripotent stem cells are characterized by less frequent contribution to ontogenesis, X-chromosome transcription activity found only for one chromatid, and high-level transcription-suppressive histone modification. A marker gene for primed pluripotent stem cells is OTX2, and marker genes for naive pluripotent stem cells are REX1 and the KLF family. Primed pluripotent stem cells form flat colonies, and naive pluripotent stem cells form dome-shaped colonies. In particular, it is preferable to use primed pluripotent stem cells for the pluripotent stem cells to be used in the present specification

Commercially available or donated cells or newly prepared cells may be used for the pluripotent stem cells to be used in the present specification. In use for the invention of the present specification, the pluripotent stem cells are preferably, but not limited to, iPS cells or ES cells.

When a commercially available product is used for the iPS cells to be used in the present specification, these products that can be used include, but are not limited to, 253G1 strain, 253G4 strain, 201B6 strain, 201B7 strain, 409B2 strain, 454E2 strain, 606A1 strain, 610B 1 strain, 648A1 strain, HiPS-RIKEN-1A strain, HiPS-RIKEN-2A strain, HiPS-RIKEN-12A strain, Nips-B2 strain, TkDN4-M strain, TkDA3-1 strain, TkDA3-2 strain, TkDA3-4 strain, TkDA3-5 strain, TkDA3-9 strain, TkDA3-20 strain, hiPSC38-2 strain, MSC-iPSC1 strain, BJ-iPSC1 strain, RPChiPS771-2, WTC-11 strain, 1231A3 strain, 1383D2 strain, 1383D6 strain, 1210B2 strain, 1201C1 strain, and 1205B2 strain.

When iPS cells for clinical use are used herein, examples of strains include, but are not limited to, QHJI01s01 strain, QHJI01s04 strain, QHJI14s03 strain, QHJI14s04 strain, Ff-l14s03 strain, Ff-l14s04 strain, and YZWI strain.

When the iPS cells to be used in the present specification are newly prepared cells, combinations of genes for initialization factors to be introduced include, but are not limited to, a combination of the OCT3/4 gene, the KLF4 gene, the SOX2 gene, and the c-Myc gene (Yu J, et al. 2007, Science, 318: 1917-20) and a combination of the OCT3/4 gene, the SOX2 gene, the LIN28 gene, and the Nanog gene (Takahashi K, et al. 2007, Cell, 131: 861-72). Any mode may be employed for introducing those genes into cells, without limitation; for example, transfection with plasmids or introduction of synthetic RNA may be employed, and proteins formed therefrom may be introduced. Alternatively, iPS cells prepared with a method involving the use of microRNA, RNA, a small-molecule compound, or the like may be used. Moreover, newly prepared clinical-grade iPS cells may be used.

When a commercially available product is used for the ES cells to be used in the present specification, commercially available products that can be used include, but are not limited to, KhES-1 strain, KhEs-2 strain, KhEs-3 strain, KhEs-4 strain, KhEs-5 strain, SEES1 strain, SEES2 strain, SEES3 strain, SEES-4 strain, SEES-5 strain, SEES-6 strain, SEES-7 strain, HUES8 strain, CyT49 strain, H1 strain, H9 strain, and HS-181 strain.

### <<Cell Aggregate>>

In the present specification, a "cell aggregate" is a massive cell population formed through cell clumping in suspension culture, and it is also referred to as a spheroid. A cell aggregate is approximately spherical in general. Cells constituting a cell aggregate are not particularly limited as long as they are one or more types of the aforementioned cells. For example, a cell aggregate composed of pluripotent stem cells such as human pluripotent stem cells or human embryonic stem cells include cells expressing a pluripotent stem cell marker and/or being positive for a pluripotent stem cell marker. A cell aggregate may be formed through microcarriers.

Pluripotent stem cell markers are gene markers specifically or excessively expressed in pluripotent stem cells, and examples thereof can include Alkaline Phosphatase, Nanog, OCT4, SOX2, TRA-1-60, c-Myc, KLF4, LIN28, SSEA-4, and SSEA-1.

Pluripotent stem cell markers can be detected with any detection method in the art. Examples of methods for detecting cell markers include, but are not limited, flow cytometry. In the case that a fluorescence-labeled antibody is used as a detection reagent in flow cytometry, a cell emitting more intense fluorescence than a negative control (isotype control or FMO control) detected is determined to be "positive" for the marker. The proportion of cells positive for a fluorescence-labeled antibody as analyzed by flow cytometry is occasionally referred to as the "positive rate." Any antibody known in the art can be used for such fluorescence-labeled antibodies, and examples thereof include, but are not limited to, antibodies labeled with fluorescein isothiocyanate (FITC), phycoerythrin (PE), and allophycocyanin (APC).

When cells constituting a cell aggregate are pluripotent stem cells, the positive rate for a pluripotent stem cell marker is preferably 80% or higher, more preferably 90% or higher, more preferably 91% or higher, more preferably 92% or higher, more preferably 93% or higher, more preferably 94% or higher, more preferably 95% or higher, more preferably 96% or higher, more preferably 97% or higher, more preferably 98% or higher, more preferably 99% or higher, and more preferably 100% or lower. Cell aggregates in which the percentage of cells expressing a pluripotent stem cell marker and/or being positive for a pluripotent stem cell marker falls within the range are highly undifferentiated and highly homogeneous cell populations.

### <<Adherent culture and medium>>

"Adherent culture," one of cell culture methods, refers to allowing cells to proliferate as a monolayer in principle with the cells adhered to an external matrix or the like such as a culture vessel. Examples of external matrices that can be used include, but are not particularly limited to, Laminin, Vitronectin, Gelatin, Collagen, and E-Cadherin chimeric antibody. A suspension culture method is a counterpart culture method to the adherent culture method. "Suspension culture" refers to allowing cells to proliferate in a suspended state in medium. In the "suspended state" herein, cells in a culture solution are not fixed on or attached to a culture vessel or the like. In a method of culture in which cells are attached to microcarriers and cultured in a suspended state in a culture solution, cells are attached to microcarriers, but a cell aggregate including microcarriers is suspended without being attached to a culture vessel. Such method of culture can be regarded as "suspension culture." In a "suspension culture method," cells are subjected to suspension culture. According to such method, in general, cells are present as a cell cluster formed through aggregation in a culture solution. In general, the cells can be cultured not only by adherent culture but also by suspension culture.

The term "medium" used herein refers to a liquid or solid substance prepared for cell culture. In principle, a medium contains components indispensable for proliferation and/or maintenance of cells over their minimum requirements. Unless otherwise stated, a liquid medium for animal cells for use in culture of cells derived from an animal is employed as medium in the present specification.

In the present specification, the term "basal medium" refers to a medium that serves as a base for media for various animal cells. Culture can be performed with the use of a basal medium by itself, and media specific to different cells for different purposes may be prepared with the addition of various culture additives. Examples of basal medium used in the present specification include, but are not limited to, BME medium, BGJb medium, CMRL1066 medium, Glasgow MEM medium, Improved MEM Zinc Option medium, IMDM medium (Iscove's Modified Dulbecco's Medium), Medium 199 medium, Eagle MEM medium, αMEM medium, DMEM medium (Dulbecco's Modified Eagle's Medium), Ham's F10 medium, Ham's F12 medium, RPMI 1640 medium, Fischer's medium, and mixed media of them (e.g., DMEM/F12 medium (Dulbecco's Modified Eagle's Medium/Nutrient Mixture F-12 Ham)). For DMEM/F12 medium, in particular, a medium obtained by mixing DMEM medium and Ham's F12 medium at a weight ratio preferably in the range of 60/40 or higher to 40/60 or lower, such as 58/42, 55/45, 52/48, 50/50, 48/52, 45/55, and 42/58, is used. In addition, other media used for culture of human iPS cells and human ES cells can be preferably used.

A medium to be used in the present invention is preferably a medium containing no serum, in other words, a serum-free medium.

In the present specification, a "culture additive" is a substance other than serum, which is added to a medium for culture. Specific examples of culture additives include, but are not limited to, L-ascorbic acid, insulin, transferrin, selenium, sodium hydrogen carbonate, growth factors, fatty acid or lipid, amino acids (e.g., non-essential amino acids), vitamins, cytokines, antioxidants, 2-mercaptoethanol, pyruvic acid, buffers, inorganic salts, and antibiotics. Insulin, transferrin, and cytokines may be naturally occurring ones separated from tissue, serum, or the like of an animal (preferably a human, mouse, rat, bovine, horse, or goat) or genetically engineered recombinant proteins. Examples of growth factors that can be used include, but are not limited to, FGF2 (Basic fibroblast growth factor-2), TGF-β1 (Transforming growth factor-β1), Activin A, IGF-1, MCP-1, IL-6, PAI, PEDF, IGFBP-2, LIF, and IGFBP-7. Examples of antibiotics that can be used include, but are not limited to, penicillin, streptomycin, and amphotericin B. Particularly preferable growth factors as culture additives of a medium to be used in the present invention are FGF2 and/or TGF-β1.

It is preferable that each medium contain a ROCK inhibitor. An example of a ROCK inhibitor is Y-27632. By adding a ROCK inhibitor into a medium, cell death in adherent culture of pluripotent stem cells can be reduced to a significant extent. In addition, the strength of a cell mass can be increased, and tolerance thereof to physical damages can be enhanced. For example, the lower limit of the concentration of a ROCK inhibitor in a medium can be 1 µM, 2 µM, 3 µM, 5 µM, 7 µM, or 10 µM, and the upper limit thereof can be 50 µM, 40 µM, 30 µM, 20 µM, or 10 µM.

When primed pluripotent stem cells are to be cultured, it is particularly preferable that each medium have a composition free of LIF. When primed pluripotent stem cells are to be cultured, it is preferable to employ a medium composition free of either or both of a GSK3 inhibitor and a MEK/ERK inhibitor. Such medium free of LIF, a GSK3 inhibitor, and a MEK/ERK inhibitor allows primed pluripotent stem cells to be cultured with the undifferentiated state maintained, without causing conversion into the naive state.

A medium to be used in the present invention can contain one or more of the culture additives. A medium to which the culture additives are to be added is typically any of the basal media, although the medium is not limited thereto.

A culture additive in the form of, for example, a solution, derivative, salt, or a mixed reagent can be added to a medium. For example, L-ascorbic acid in the form of a derivative, such as magnesium ascorbyl-2-phosphate, may be added to a medium, and selenium in the form of a selenite (such as sodium selenite) may be added to a medium. Insulin, transferrin, and selenium in the form of an ITS reagent (insulin-transferrin-selenium) can be added to a medium. A commercially available medium to which at least one substance selected from among L-ascorbic acid, insulin, transferrin, selenium, and sodium hydrogen carbonate may be used. Examples of commercially available media to which insulin and transferrin have been added include CHO-S-SFM II (Life Technologies Japan Ltd.), Hybridoma-SFM (Life Technologies Japan Ltd.), eRDF Dry Powdered Media (Life Technologies Japan Ltd.), UltraCULTURE^{™} (BioWhittaker), UltraDOMA^{™} (BioWhittaker), UltraCHO^{™} (BioWhittaker), UltraMDCK^{™} (BioWhittaker), STEMPRO^{®} hESC SFM (Life Technologies Japan Ltd.), Essential 8^{™} (Life Technologies Japan Ltd.), StemFit^{®} AK02N (Ajinomoto Co., Inc.), mTeSR1 (VERITAS Corporation), TeSR2 (VERITAS Corporation), ReproMed (ReproCELL, Inc.), and StemScale (Thermo Fisher Scientific K.K.).

The most preferable medium to be used in the present invention is a serum-free medium containing L-ascorbic acid, insulin, transferrin, selenium, sodium hydrogen carbonate, and at least one growth factor. A serum-free DMEM/F 12 medium containing L-ascorbic acid, insulin, transferrin, selenium, sodium hydrogen carbonate, and at least one growth factor (preferably FGF2 and TGF-β1) is particularly preferable.

### <<PKCβ inhibitor>>

In the present specification, the term "protein kinase C β (PKCβ) inhibitor" means a substance that inhibits or suppresses the activity of PKCβ. Protein kinases each have a catalytic region in the C-terminal side and a regulatory region in the N-terminal side. The catalytic region is composed of a sequence that recognizes phosphorylated residues on a substrate protein and a sequence that forms an ATP/Mg²⁺ bond. The regulatory region is composed of C1 and C2 domains.

PKC includes PKCα, PKCβI, PKCβII, and PKCγ as conventional isozymes. PKC includes PKCδ, PKCε, PKCθ, and PKCη as novel isozymes, and PKCζ, PKCλ, and PKCµ as atypical isozymes.

In the present specification, the term PKCβ means both of those PKCβI and PKCβII, or one of PKCβI and PKCβII. In the present specification, the term PKCβ inhibitor means a substance that inhibits at least PKCβI and/or PKCβII among those conventional, novel, and atypical isozymes. That is, the term PKCβ inhibitor means any of a substance that inhibits or suppresses only the activity of PKCβI, a substance that inhibits or suppresses only the activity of PKCβII, and a substance that inhibits or suppresses the activities of PKCβI and PKCβII.

The PKCβ inhibitor may be a substance that specifically inhibits or suppresses only the activity of PKCβ or a substance that inhibits or suppresses the activity of another isozyme in addition to that of PKCβI or PKCβII. For example, the PKCβ inhibitor may be a substance that inhibits or suppresses the activities of all the aforementioned conventional, novel, and atypical isozymes including PKCβI and PKCβII. The PKCβ inhibitor may be a substance that inhibits or suppresses the activities of the conventional isozymes PKCα and PKCγ in addition to those of PKCβI and PKCβII. Moreover, the PKCβ inhibitor may be a substance that inhibits or suppresses the activities of the novel isozymes PKCδ, PKCε, PKCθ, and PKCη in addition to those of PKCβI and PKCβII.

Examples of the PKCβ inhibitor include a compound that directly or indirectly acts on PKCβ, an antisense nucleic acid for a gene encoding PKCβ, an RNA-interference-inducible nucleic acid (e.g., siRNA), a dominant-negative mutant, and an expression vector for any thereof.

An example of the PKCβ inhibitor can be a compound having the following structural formula (Formula [I]) or a salt thereof:

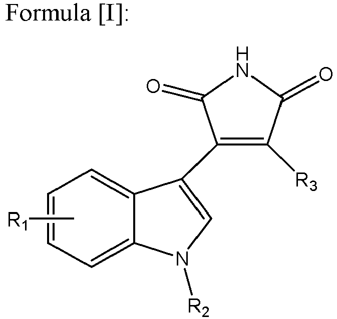

In Formula [I],
R₁ is a hydrogen atom or an alkoxy group having 1 to 3 carbon atoms (preferably a methoxy group);
R₂ is a hydrogen atom, an alkyl group having 1 to 3 carbon atoms (preferably a methyl group), or an alkyl group having 1 to 3 carbon atoms substituted with -N(R_{A})₂ (preferably -(CH₂)₃-N(CH₃)₂);
R_{A} is independently an ethyl group or a methyl group (preferably a methyl group);
R₃ is a group represented by: 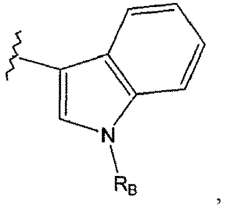 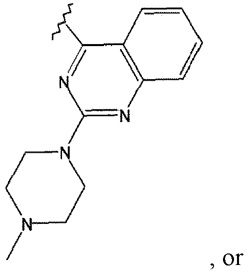 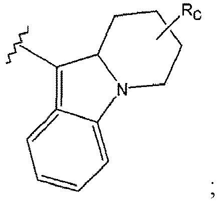
R_{B} is a hydrogen atom, an alkyl group having 2 to 4 carbon atoms substituted with -S-C(=NH)(-NH₂) (preferably -(CH₂)₃-S-C(=NH)(-NH₂)), or a group represented by: 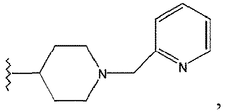
R₂ and R_{B} may together form a divalent group represented by: 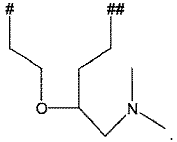 wherein
   a symbol "#" indicates binding of R₂ to a site of binding and a symbol "##" indicates binding of Rato a site of binding,
   a steric configuration of asymmetrical carbon included in the divalent group is preferably represented by, but is not particularly limited to: 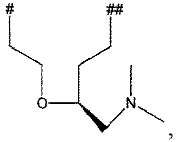
   Rc is an alkyl group having 1 to 3 carbon atoms substituted with -N(R_{D})₂ (preferably -(CH₂)-N(CH₃)₂), and
   R_{D} is independently an ethyl or methyl group (preferably a methyl group).

Examples of a salt of a compound represented by Formula I include a hydrochloride and a sulfate.

Specific examples of the PKCβ inhibitor having the above structural formula [Formula I] include a compound selected from the group consisting of Go6983, GF109203X, LY-333531, Enzastaurin, Sotrastaurin, Ro-31-8220-mesylate, Ro-32-0432-hydrochloride, Go6976, Rottlerin, Midostaurin, Daphnetin, Dequalinium Chloride, Baicalein, Quercetin, Luteolin, Bisindolylmaleimide II, Calphostin C, Chelerythrine chloride, L-threo-Dihydrosphingosine, and Melittin. Among the PKCβ inhibitors having the above structural formula, a compound selected from the group consisting of Go6983, GF109203X, and LY-333531 is preferably used.

The structural formula of Go6983 (3-[1-[3-(dimethylamino)propyl]-5-methoxy-1H-indol-3-yl]-4-(1H-indol-3-yl)-1H-pyrrole-2,5-dione) is shown in the following.

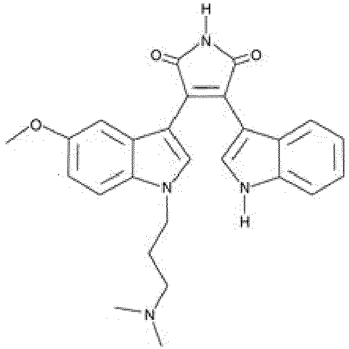

The structural formula of GF109203X (2-[1-(3-dimethylaminopropyl)indol-3-yl]-3-(indol-3-yl)maleimide) is shown in the following.

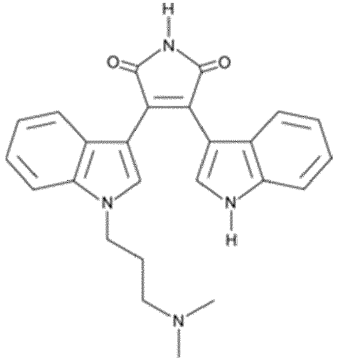

The structural formula of LY-333531 ((9S)-[(dimethylamino)methyl]-6,7,10,11-tetrahydro-9H,18H-5,21:12,17-dimethenodibenzo[e,k]pyrrolo[3,4-h][1,4,13]oxsadiazacyclohexadecine-18,20(19H)-dione, monohydroxychloride) is shown in the following.

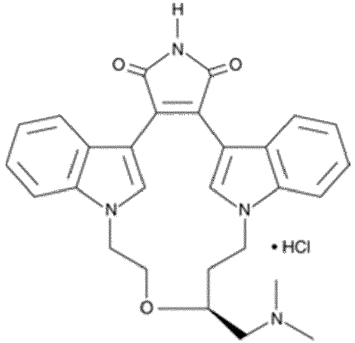

The structural formula of Enzastaurin (3-(1-methylindol-3-yl)-4-[1-[1-(pyridin-2-ylmethyl)piperidin-4-yl]indol-3-yl]pyrrole-2,5-dione) is shown in the following.

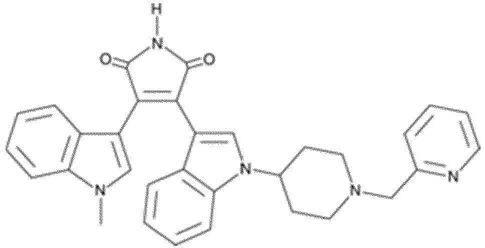

The structural formula of Sotrastaurin (3-(1H-indol-3-yl)-4-(2-(4-methylpiperazin-1-yl)quinazolin-4-yl)-1H-pyrrole-2,5-dione) is shown in the following.

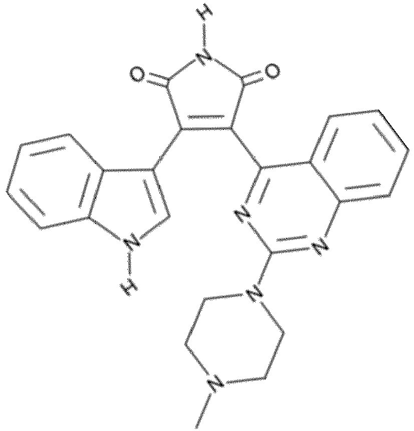

The structural formula of Ro-31-8220-mesylate (3-[3-[2,5-dihydro-4-(1-methyl-1H-indol-3-yl)-2,5-dioxo-1H-pyrrole-3-yl]-1H-indol-1-yl]propyl carbamimidothioate mesylate) is shown in the following.

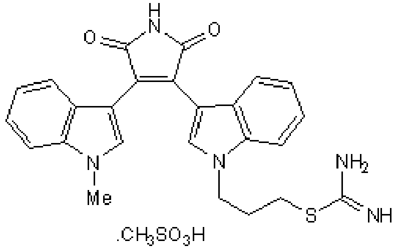

The structural formula of Ro-32-0432-hydrochloride (3-[(8S)-8-[(dimethylamino)methyl]-6,7,8,9-tetrahydropyrido[1,2-a]indol-10-yl]-4-(1-methyl-1H-indol-3-yl)-1H-pyrrole-2,5-dione hydrochloride) is shown in the following.

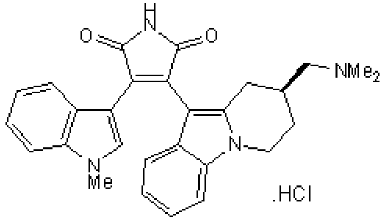

### <<TNKS inhibitor>>

In the present specification, the term "tankyrase (TNKS) inhibitor" means a substance that inhibits or suppresses the activity of tankyrase. Tankyrase belongs to the poly(ADP-ribose) polymerase (PARP) family to poly(ADP-ribosylate) a target protein, and tankyrase 1 (tankyrase-1/PARP-5a) and tankyrase 2 (tankyrase-2/PARP-5b) are known. Tankyrase is known to have a function to promote the telomere elongation by telomerase through poly(ADP-ribosylation) of the telomere protein TRF1 to separate it from a telomere.

In the present specification, the term TNKS means both of those tankyrase 1 and tankyrase 2, or one of tankyrase 1 and tankyrase 2. In the present specification, the term TNKS inhibitor means a substance that inhibits tankyrase 1 and/or tankyrase 2. That is, the term TNKS inhibitor means any of a substance that inhibits or suppresses only the activity of tankyrase 1, a substance that inhibits or suppresses only the activity of tankyrase 2, and a substance that inhibits or suppresses the activities of tankyrase 1 and tankyrase 2.

Examples of the TNKS inhibitor include a compound that directly or indirectly acts on TNKS, an antisense nucleic acid for a gene encoding TNKS, an RNA-interference-inducible nucleic acid (e.g., siRNA), a dominant-negative mutant, and an expression vector for any thereof.

An example of the TNKS inhibitor can be a compound selected from the group consisting of IWR-1-endo, XAV939, G007-LK, G244-LM, MSC2504877, and WIKI4. Among the TNKS inhibitors, IWR-1-endo and/or XAV939 is particularly preferably used.

The structural formula of IWR-1-endo (4-(1,3,3a,4,7,7a-hexahydro-1,3-dioxo-4,7-methano-2H-isoindol-2-yl)-N-8-quinolinyl-benzamide) is shown in the following.

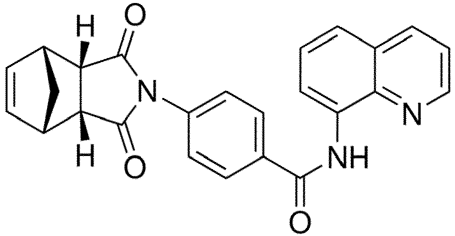

The structural formula of XAV939 (3,5,7,8-tetrahydro-2-[4-(trifluoromethyl)phenyl]-4H-thiopyrano[4,3-d]pyrimidin-4-one) is shown in the following.

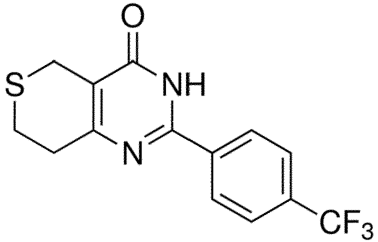

The structural formula of G007-LK (4-[5-[(1E)-2-[4-(2-chlorophenyl)-5-[5-(methylsulfonyl)-2-pyridinyl]-4H-1,2,4-thiazol-3-yl]ethenyl]-1,3,4-oxsadiazol-2-yl]-benzonitrile) is shown in the following.

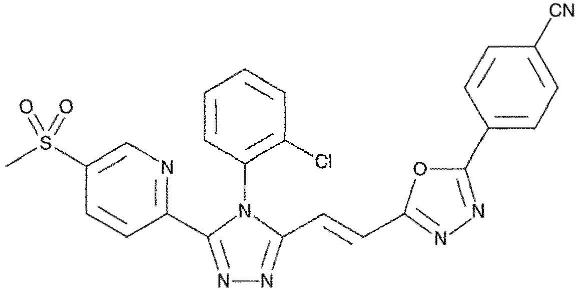

The structural formula of G244-LM (3,5,7,8-tetrahydro-2-[4-[2-(methylsulfonyl)phenyl]-1-piperazinyl]-4H-thiopyrano[4,3-d]pyrimidin-4-one) is shown in the following.

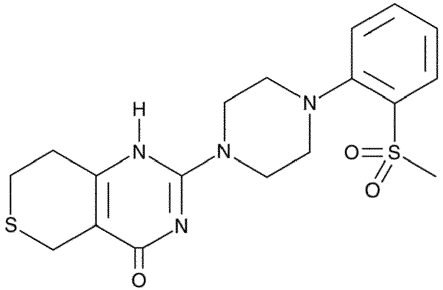

The structural formula of WIKI4 (2-[3-[[4-(4-methoxyphenyl)-5-(4-pyridinyl)-4H-1,2,4-thiazol-3-yl]thio]propyl]-1H-benzo[de]isoquinoline-1,3(2H)-dione) is shown in the following.

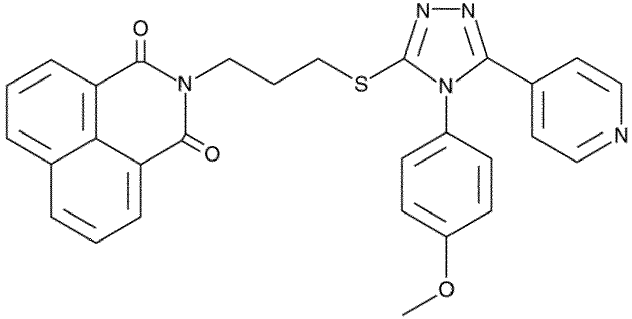

### 1-3. An inhibitor of the death of pluripotent stem cells for adherent culture

In the method for producing a pluripotent stem cell population according to the present invention, a pluripotent stem cell population is first subjected to adherent culture and then to suspension culture. In particular, a liquid medium used for adherent culture contains, as active ingredients, the PKCβ inhibitor and the TNKS inhibitor defined in 1-2. above. Accordingly, an aspect of the present invention concerns an inhibitor of the death of pluripotent stem cells for adherent culture comprising, as active ingredients, a PKCβ inhibitor and a TNKS inhibitor.

The cell death inhibitor may comprise one PKCβ inhibitor or a combination of two or more different PKCβ inhibitors. When the inhibitor of the death of pluripotent stem cells for adherent culture is a composition, the concentration of the PKCβ inhibitor in the composition can be determined to be within a desirable range (described below) when it is added to a medium.

The inhibitor of the death of pluripotent stem cells for adherent culture may comprise one TNKS inhibitor or a combination of two or more different TNKS inhibitors. When the inhibitor of the death of pluripotent stem cells for adherent culture is a composition, the concentration of the TNKS inhibitor in the composition can be determined to be within a desirable range (described below) when it is added to a medium.

The PKCβ inhibitor and the TNKS inhibitor as active ingredients can be added to a medium in combination with a carrier. The carrier contains a solvent and/or an excipient.

Examples of the solvent include water, buffers (including PBS), physiological saline, and organic solvents (DMSO, DMF, xylene, and lower alcohols).

Examples of the excipient include antibiotics, buffers, thickeners, coloring agents, stabilizers, surfactants, emulsifying agents, antiseptics, preservatives, and antioxidants. Applicable antibiotics include, but are not limited to, penicillin, streptomycin, and amphotericin B. Examples of buffers include phosphate buffer, Tris-hydrochloric acid buffer, and glycine buffer. Examples of thickeners include gelatin and polysaccharides. Examples of coloring agents include phenol red. Examples of stabilizers include albumin, dextran, methylcellulose, and gelatin. Examples of surfactants include cholesterol, alkyl glycoside, alkyl polyglucoside, alkyl monoglyceryl ether, glucoside, maltoside, neopentyl glycols, polyoxyethylene glycols, thioglucoside, thio maltoside, peptide, saponin, phospholipid, fatty acid sorbitan ester, and fatty acid diethanolamide. Examples of emulsifying agents include glycerin fatty acid ester, sorbitan fatty acid ester, propylene glycol fatty acid ester, and sucrose fatty acid ester. Examples of antiseptics include aminoethylsulfonic acid, benzoic acid, sodium benzoate, ethanol, sodium edetate, agar, dl-camphor, citric acid, sodium citrate, salicylic acid, sodium salicylate, phenyl salicylate, dibutylhydroxytoluene, sorbic acid, potassium sorbate, nitrogen, dehydroacetic acid, sodium dehydroacetate, 2-naphthol, white soft sugar, honey, isobutyl para-hydroxybenzoate, isopropyl para-hydroxybenzoate, ethyl para-hydroxybenzoate, butyl para-hydroxybenzoate, propyl para-hydroxybenzoate, methyl para-hydroxybenzoate, 1-menthol, and Eucalyptus oil. Examples of preservatives include benzoic acid, sodium benzoate, ethanol, sodium edetate, dried sodium sulfite, citric acid, glycerin, salicylic acid, sodium salicylate, dibutylhydroxytoluene, D-sorbitol, sorbic acid, potassium sorbate, sodium dehydroacetate, isobutyl para-hydroxybenzoate, isopropyl para-hydroxybenzoate, ethyl para-hydroxybenzoate, butyl para-hydroxybenzoate, propyl para-hydroxybenzoate, methyl para-hydroxybenzoate, propylene glycol, and phosphoric acid. Examples of antioxidants include citric acid, citric acid derivatives, vitamin C and derivatives thereof, lycopene, vitamin A, carotenoids, vitamin B and derivatives thereof, flavonoids, polyphenols, glutathione, selenium, sodium thiosulfate, vitamin E and derivatives thereof, α-lipoic acid and derivatives thereof, pycnogenol, flavangenol, superoxide dismutase (SOD), glutathione peroxidase, glutathione-S-transferase, glutathione reductase, catalase, ascorbate peroxidase, and mixtures of them.

An inhibitor of the death of pluripotent stem cells for adherent culture may contain one or more growth factors. Examples of the growth factors include FGF2 and TGF-β1.

### 1-4. Effects

According to the method for preparing a pluripotent stem cell population of the present invention, pluripotent stem cells are first subjected to adherent culture, the pluripotent stem cells are then subjected to suspension culture, and the death of pluripotent stem cells caused thereby can be inhibited. When the death of pluripotent stem cells is inhibited, a large number of pluripotent stem cells can be maintained when the pluripotent stem cells are subjected to suspension culture after adherent culture. That is, the number of cells remaining after adherent culture in the presence of a PKCβ inhibitor and a TNKS inhibitor followed by suspension culture is compared with the number of cells remaining after adherent culture in the absence of either or both of a PKCβ inhibitor and a TNKS inhibitor followed by suspension culture under the same conditions. The results of comparison demonstrate that a larger number of cells remain when adherent culture is performed in the presence of the PKCβ inhibitor and the TNKS inhibitor.

By applying the method for preparing a pluripotent stem cell population according to the present invention, the cell death occurring at the time of suspension culture can be inhibited. Thus, a pluripotent stem cell population can be efficiently produced by suspension culture. Specifically, a pluripotent stem cell population comprising a desirable number of cells can be produced within a relatively short period of time. This can reduce a cost necessary for production of a pluripotent stem cell population to a significant extent.

### 1-5. Culture step

The method according to the present invention comprises a step of adherent culture and a subsequent step of suspension culture. The method according to the present invention may comprise a step of collection. These steps will be described in the following.

### 1-5-1. A step of adherent culture

In the "step of adherent culture," a cell population before the step of suspension culture is cultured to proliferate while maintaining an undifferentiated state. Adherent culture can be performed by employing a method of animal cell culture known in the art. For example, a method of adherent culture in which cells are cultured while being adhered to a culture substrate, such as a vessel and a carrier, may be adopted.

### (Cells)

The cells to be used in the present step can be subjected to adherent culture, and such cells can aggregate in the step of suspension culture described below. As described in the subsection "Culture and Medium" in "1-2. Definition of Terms" above, animal cells are preferable, and human cells are more preferable. Cells are pluripotent stem cells, and pluripotent stem cells such as iPS cells and ES cells are particularly preferable. The pluripotent stem cells to be used in the present step may be a type of cells or a cell population comprising plural types of cells (i.e., a pluripotent stem cell population). When the pluripotent stem cells constitute a pluripotent stem cell population, the percentage (proportion) of cells expressing a pluripotent stem cell marker (e.g., OCT4, SOX2, or Nanog) and/or being positive for a pluripotent stem cell marker in the population is, for example, 90% or higher, 91% or higher, 92% or higher, 93% or higher, 94% or higher, 95% or higher, 96% or higher, 97% or higher, 98% or higher, 99% or higher, or 100% or lower.

### (Culture vessel)

A culture vessel used for adherent culture is not particularly limited. A vessel with an inner surface that is not processed to suppress protein adsorption thereto is preferable, and a vessel with an external matrix that can be coated is preferable. The shape of the culture vessel is not particularly limited, and examples of the culture vessel include dish-like, flask-like, well-like, bag-like, and spinner-flask-like culture vessels. For example, a cell culture petri-dish (Sumitomo Bakelite Co., Ltd.) can be used as a culture vessel.

The capacity of the culture vessel to be used is not limited, and an appropriate capacity can be selected; however, it is preferable that the lower limit of the area of the bottom of a part to contain medium in plan view be 0.32 cm², 0.65 cm², 1.9 cm², 3.0 cm², 3.5 cm², 9.0 cm², or 9.6 cm², and the upper limit thereof be 1000 cm², 500 cm², 300 cm², 150 cm², 75 cm², 55 cm², 25 cm², 21 cm², 10 cm², or 3.5 cm².

The capacity of the culture vessel to be used is not limited, and an appropriate capacity can be selected; however, it is preferable that the lower limit of the volume that can accommodate a medium and perform culture therein be 1 ml, 2 ml, 4 ml, 10 ml, 20 ml, 30 ml, 50 ml, 100 ml, 200 ml, 500 ml, 1 l, 3 l, 5 l, 10 l, or 20 1, and the upper limit thereof be 100 l, 50 l, 20 l, 10 l, 5 l, 3 l, 1 l, 500 m 1, 200 m 1, 100 m 1, 50 m 1, or 30 ml.

### (Medium)

A medium used for adherent culture is prepared by supplementing the basal medium described in "1-2. Definition of Terms" above with a PKCβ inhibitor and a TNKS inhibitor. A medium is not limited, provided that the medium contains a PKCβ inhibitor and a TNKS inhibitor, and pluripotent stem cells can be proliferated and/or be maintained therein. In particular, use of a medium not containing a leukemia inhibitory factor is preferable. The PKCβ inhibitor may be one PKCβ inhibitor or a combination of two or more different PKCβ inhibitors. The lower limit of the concentration of the PKCβ inhibitor is not particularly limited, and it can be determined within a range that would not cause cell death.

For example, the final concentration of the PKCβ inhibitor in the liquid medium can be 25 nM or more, 30 nM or more, 50 nM or more, 80 nM or more, 100 nM or more, 150 nM or more, 200 nM or more, 500 nM or more, or 700 nM or more.

The upper limit of the concentration of the PKCβ inhibitor is not particularly limited, and it can be determined in accordance with, for example, the range in which the PKCβ inhibitor would not cause cell death, the range in which the PKCβ inhibitor would not be toxic on pluripotent stem cells, and the solubility of the PKCβ inhibitor.

For example, the final concentration of the PKCβ inhibitor in the liquid medium can be 15 µM or less, 10 µM or less, 5 µM or less, 3 µM or less, or 1 µM or less.

The TNKS inhibitor may be one TNKS inhibitor or a combination of two or more different TNKS inhibitors. The lower limit of the concentration of the TNKS inhibitor is not particularly limited, and it can be determined in accordance with the range in which the TNKS inhibitor would not cause cell death.

For example, the final concentration of the TNKS inhibitor in the liquid medium can be 90 nM or more, 100 nM or more, 150 nM or more, 200 nM or more, 300 nM or more, 400 nM or more, 500 nM or more, 600 nM or more, 700 nM or more, 800 nM or more, or 900 nM or more.

The upper limit of the concentration of the TNKS inhibitor is not limited, and it can be determined in accordance with, for example, the range in which the TNKS inhibitor would not cause cell death, the range in which the TNKS inhibitor would not be toxic on pluripotent stem cells, and the solubility of the TNKS inhibitor.

For example, the final concentration of the TNKS inhibitor in the liquid medium can be 40 µM or less, 35 µM or less, 30 µM or less, 15 µM or less, 10 µM or less, 5 µM or less, 3 µM or less, 1.5 µM or less, or 1 µM or less.

The lower limit of the ratio of the concentrations of the PKCβ inhibitor to that of the TNKS inhibitor in the liquid medium (molar concentration) is not particularly limited, and such lower limit can be, for example, 167:1, 111:1, 56:1, 33:1, 11:1, 7.8:1, 5.6:1, 2.2:1, 1.7:1, or 1.1:1. The upper limit of the ratio of the concentrations of the PKCβ inhibitor to that of the TNKS inhibitor in the liquid medium (molar concentration) is not particularly limited, and such upper limit can be, for example, 1:1600, 1:1400, 1:1200, 1:600, 1:400, 1:200, 1:120, 1:60, 1:40, 1:36, 1:32, 1:28, 1:24, 1:20, 1:16, 1:12, 1:8, 1:6, or 1:4. The ratio of the concentrations of the PKCβ inhibitor to that of the TNKS inhibitor in the liquid medium (molar concentration) is not particularly limited, and such ratio can be, for example, 167:1 to 1:1600. The ratio of the concentrations of the PKCβ inhibitor to that of the TNKS inhibitor in the liquid medium (molar concentration) is preferably 111:1 to 1:1600, 56:1 to 1:1600, 33:1 to 1:1600, 11:1 to 1:1600, 7.8:1 to 1:1600, 5.6:1 to 1:1600, 2.2:1 to 1:1600, 1.7:1 to 1:1600, or 1.1:1 to 1:1600. Moreover, the ratio of the concentrations of the PKCβ inhibitor to that of the TNKS inhibitor in the liquid medium (molar concentration) is preferably 167:1 to 1:1400, 167:1 to 1:1200, 167:1 to 1:600, 167:1 to 1:400, 167:1 to 1:200, 167:1 to 1:120, 167:1 to 1:60, 167:1 to 1:40, 167:1 to 1:36, 167:1 to 1:32, 167:1 to 1:28, 167:1 to 1:24, 167:1 to 1:20, 167:1 to 1:16, 167:1 to 1:12, 167:1 to 1:8, 167:1 to 1:6, or 167:1 to 1:4.

A PKCβ inhibitor and a TNKS inhibitor may be added in any manner without limitation, as long as the concentrations of a PKCβ inhibitor and a TNKS inhibitor in the medium at the beginning of the present step fall within the above ranges. For example, the medium may be prepared by directly adding one or more PKCβ inhibitors and TNKS inhibitors to the medium, so that each concentration in total falls within the corresponding concentration range shown above.

The amount of a medium or a culture solution used for adherent culture may be adequately adjusted in accordance with a culture vessel to be used. The amount is preferably adjusted to bring the liquid level from the bottom of the culture vessel to 2 mm. When a 15-cm dish (an area of the dish bottom in plan view: 150 cm²) is used, for example, such amount can be 30 ml.

### (Seeding Density)

The density of cells to be seeded (seeding density) in a fresh medium in adherent culture can be appropriately adjusted in view of culture time, the condition of cells after culture, and the number of cells needed after culture. In general, the lower limit is, for example, 0.1 × 10⁴ cells/cm², 1 × 10⁴ cells/cm², or 2 × 10⁴ cells/cm², and the upper limit is, for example, 20 × 10⁴ cells/cm² or 10 × 10⁵ cells/cm², although the lower limit and upper limit are not limited thereto.

### (Culture Conditions)

There is no limitation to the culture conditions including culture temperature, duration, and CO₂ concentration. Culture can be performed with any of conventional methods in the art. The lower limit of the culture temperature can be, for example, 20°C or 35°C, and the upper limit thereof can be 45°C or 40°C, with the culture temperature of 37°C being preferable. The lower limit of the duration of a passage is 0.5 hours or 6 hours, and the upper limit thereof is 8 days, 120 hours, 96 hours, 72 hours, or 48 hours. The lower limit of CO₂ concentration in culture can be 0.5%, 1%, 2%, 3%, 4%, or 4.5%, and the upper limit thereof can be 10% or 5.5%, with the CO₂ concentration of 5% being preferable. The CO₂ concentration in culture is not necessarily constant, and it may be modified or changed during culture. The lower limit of the O₂ concentration in culture can be, for example, 3% or 5%, and the upper limit thereof can be 21% or 20%, with the O₂ concentration of 21% being preferable. In adherent culture, medium exchange can be performed at an appropriate frequency. While the frequency of medium exchange varies among cell types to be cultured, medium exchange can be performed, for example, one or more times per 5 days, one or more times per 4 days, one or more times per 3 days, one or more times per 2 days, one or more times per day, or two or more times per day. Alternatively, medium exchange may be performed continuously by the perfusion system. Medium exchange can be performed in such a manner that cells are collected with the same method as in the step of collection described below, a fresh medium is then added, the cell aggregate is gently dispersed, and cultured may then be performed again. When medium exchange is performed by the perfusion system, cells may be separated from the medium with the use of a filter to retain the cells in the culture system, and medium exchange may then be performed. Alternatively, culture may be continued, the culture solution from which cells had been separated using a filter or the like may be continuously suctioned from a vessel, and fresh media may be continuously added. The frequency of and method or the like for medium exchange are not limited to the frequency and the method described above, and optimal frequency and method can be appropriately employed.

### (Culture Method)

The flowing state of a medium in adherent culture is not limited. Specifically, adherent culture may be performed by static culture or flow culture.

"Static culture" refers to culture in which a medium is left to stand in a culture vessel. The static culture is typically employed for adherent culture. "Flow culture" refers to culture in which a medium is allowed to flow. Adherent culture may be performed by allowing cells to adhere to microcarriers or the like while the flowing state of the medium is maintained.

In the step of adherent culture, the number of cells attained by proliferation can be determined as desired. The target number of cells and the targeted cell conditions can be adequately determined in accordance with the types of cells to be cultured, the purpose of cell aggregation, the types of media, and culture conditions. For example, the lower limit of an extent of cell proliferation may be 10%, 20%, 30%, 40%, or 50% in terms of the percentage of the cells occupying the culture area in the culture vessel, although the lower limit is not limited thereto. The upper limit thereof may be 100%, 90%m 80%, 70%, or 60%. It is particularly preferable that cells be grown to occupy at least 70% to 80% of the culture area in the culture vessel.

In the step of adherent culture, a part of pluripotent stem cells during culture can be taken out to examine as to whether or not the undifferentiated state is maintained. For example, whether or not the undifferentiated state is maintained can be determined by measuring the expression level of a pluripotent stem cell marker expressed on the pluripotent stem cells taken out during culture. Examples of pluripotent stem cell markers include Alkaline Phosphatase, NANOG, OCT4, SOX2, TRA-1-60, c-Myc, KLF4, LIN28, SSEA-4, and SSEA-1. As described above, an example of a method for detecting these pluripotent stem cell markers is flow cytometry.

When the positive rate of pluripotent stem cells taken out during culture for a pluripotent stem cell marker is preferably 80% or higher, more preferably 90% or higher, more preferably 91% or higher, more preferably 92% or higher, more preferably 93% or higher, more preferably 94% or higher, more preferably 95% or higher, more preferably 96% or higher, more preferably 97% or higher, more preferably 98% or higher, more preferably 99% or higher, or more preferably 100%, such pluripotent stem cells can be determined to maintain the undifferentiated state.

In the present step, whether or not the undifferentiated state is maintained can be determined by measuring the expression levels of three germ layer markers (endodermal cell marker, mesodermal cell marker, and ectodermal cell marker) in pluripotent stem cells taken out during culture. When all of the positive rates for the endodermal cell marker, the mesodermal cell marker, and the ectodermal cell marker are preferably 20% or lower, more preferably 10% or lower, more preferably 9% or lower, more preferably 8% or lower, more preferably 7% or lower, more preferably 6% or lower, more preferably 5% or lower, more preferably 4% or lower, more preferably 3% or lower, more preferably 2% or lower, more preferably 1% or lower, or more preferably below the detection limit, specifically, such pluripotent stem cells can be determined to maintain the undifferentiated state.

The endodermal cell marker is a gene specific to endodermal cells, and examples thereof include SOX17, FOXA2, CXCR4, AFP, GATA4, and EOMES. Endodermal cells differentiate to form tissue of an organ such as the gastrointestinal tract, the lung, the thyroid, the pancreas, and the liver; cells of secretory glands opening in the gastrointestinal tract; the peritoneum, the pleura, the larynx, eustachian tubes, the trachea, the bronchi, the urinary tract (the bladder, most part of the urethra, part of the ureter), and others.

The mesodermal cell marker is a gene specific to mesodermal cells, and examples thereof include TBXT (BRACHYURY), MESP1, MESP2, FOXF1, HAND1, EVX1, IRX3, CDX2, TBX6, MIXL1, ISL1, SNAI2, FOXC1, and PDGFRα. Mesodermal cells differentiate to form the celom and the mesothelium lining it, muscles, the skeleton, the dermis, connective tissues, the heart, blood vessels (including vascular endothelia), blood (including blood cells), lymphatic vessels, the spleen, the kidney, the ureter, the gonad (testis, uterus, gonad epithelium), and others.

The ectodermal cell marker is a gene specific to ectodermal cells, and examples thereof include FGF5, NESTIN, SOX1, and PAX6. Ectodermal cells differentiate to form the epidermis, the epithelium of the end of the male urethra, hair, nails, skin grands (including mammary glands and sweat glands), sense organs (including the oral cavity, the pharynx, the nose, and the epithelium of the end of the rectum, and salivary glands), the lenses, the peripheral nervous system, and others. A part of the ectoderm invaginates like a groove to form a neural tube in the developmental process, which in turn serves as the origin of neurons in the central nervous system, such as the brain and the spinal cord, and melanocytes.

The expression levels of those three germ layer markers (the endodermal cell marker, the mesodermal cell marker, and the ectodermal cell marker) can be measured with any detection method in the art. Examples of methods for measuring the expression levels of the three germ layer markers (the endodermal cell marker, the mesodermal cell marker, and the ectodermal cell marker) include, but are not limited to, quantitative real-time PCR analysis, an RNA-Seq method, Northern hybridization, and a hybridization method using a DNA array. In quantitative real-time PCR analysis, the expression level of a marker gene as a target of measurement is converted into a relative expression level to the expression level of an internal standard gene, and the expression level of the marker gene can be evaluated on the basis of the relative expression level. Examples of the internal standard gene can include a glyceraldehyde-3-phosphate dehydrogenase (GAPDH) gene and a β-actin (ACTB) gene.

### (Treatment after the step of adherent culture)

After the step of adherent culture described above, a step of collecting pluripotent stem cells may be performed, according to need. In the step of collecting pluripotent stem cells, pluripotent stem cells are separated from a culture solution in accordance with a conventional technique, and the separated pluripotent stem cells are collected. In such a case, it is preferable that pluripotent stem cells be collected as single cells by means of detachment or dispersion from the external matrix or adjacent pluripotent stem cells. Specific methods therefor will be described in detail in the step of collection below. The collected cells can be subjected to the next step directly, or after washing with a buffer (including PBS buffer), physiological saline, or a medium (a medium to be used in the next step or a basal medium being preferable), according to need.

### 1-5-2. A step of suspension culture following the step of adherent culture

In the "step of suspension culture" following the "step of adherent culture" described above, a pluripotent stem cell population obtained after the step of adherent culture is subjected to suspension culture, cells may be allowed to proliferate while maintaining the undifferentiated state, or the cells may be induced to differentiate without maintaining the undifferentiated state. The pluripotent stem cells subjected to adherent culture in a medium comprising the PKCβ inhibitor and the TNKS inhibitor may be subjected to suspension culture. Thus, the number of cells when the present step was initiated can be maintained at a sufficient level, and the production efficiency in the subsequent procedure can be improved.

The method of cell culture in the present step is fundamentally equivalent to the culture method described in "1-5-1. A step of adherent culture" above. Accordingly, description on matters in common with the method described above for the step of adherent culture is omitted, and only matters characteristic to the present step will be described in detail in the following.

### (Cells)

The cells to be used in the present step are prepared after the step of adherent culture. As described in the step of adherent culture, the cells are pluripotent stem cells, and pluripotent stem cells, such as iPS cells and ES cells, are particularly preferable. When cells are seeded in a medium, cells are preferably in the state of single cells.

### (Culture Vessel)

A culture vessel with the inner surface to which cells are less adhesive is preferably used for suspension culture. An example of the vessel with the inner surface to which cells are less adhesive include a plate subjected to hydrophilic surface treatment with a biocompatible substance. For example, a Nunclon^{™} Sphera (Thermo Fisher Scientific K.K.) can be used as the culture vessel.

### (Medium)

In the step of suspension culture, culture may be initiated in a medium that does not contain either or both the PKCβ inhibitor and the TNKS inhibitor, or culture may be initiated in a medium that contains both the PKCβ inhibitor and the TNKS inhibitor. Alternatively, the step of suspension culture may be initiated in a medium that does not contain either or both the PKCβ inhibitor and the TNKS inhibitor, the medium may be exchanged with a medium that contains both the PKCβ inhibitor and the TNKS inhibitor, and subsequent culture may be performed therein. When culture is initiated in a medium that does not contain either or both the PKCβ inhibitor and the TNKS inhibitor, a culture solution used in adherent culture may be contaminated with the PKCβ inhibitor and the TNKS inhibitor included in the culture solution when separating pluripotent stem cells from the culture solution. In suspension culture, however, a medium that does not contain either or both the PKCβ inhibitor and the TNKS inhibitor is used.

A type of the medium is not particularly limited, provided that the cells described in "1-2. Definition of Terms" above can be proliferated and/or maintained therein. A medium used in the present step may be supplemented with particular additives to allow cells to differentiate without maintaining the cells.

The volume of a medium or a culture solution used for suspension culture can be appropriately adjusted in accordance with the culture vessel to be used. When a 12-well plate (the area of the well bottom per well in plan view: 3.5 cm²) is used (the culture vessel is not limited thereto), for example, the volume per well can be 0.5 ml or more, 1.5 ml or less, and preferably approximately 1.3 ml. When a 6-well plate (the area of the well bottom per well in plan view: 9.6 cm²) is used, the volume per well can be 1.5 ml or more, 2 ml or more, or 3 ml or more to 6.0 ml or less, 5 ml or less, or 4 ml or less. When a 125-ml Erlenmeyer flask (an Erlenmeyer flask having a capacity of 125 ml) is used, the lower limit of the volume per vessel can be 10 ml, 15 ml, 20 ml, 25 ml, or 30 ml, and the upper limit thereof can be 50 ml, 45 ml, or 40 ml. When a 500-ml Erlenmeyer flask is used, the lower limit of the volume per vessel can be 100 ml, 105 ml, 110 ml, 115 ml, or 120 ml, and the upper limit thereof can be 150 ml, 145 ml, 140 ml, 135 ml, 130 ml, or 125 ml. When a 1000-ml Erlenmeyer flask is used, the lower limit of the volume per vessel can be 250 ml, 260 ml, 270 ml, 280 ml, or 290 ml, and the upper limit thereof can be 350 ml, 340 ml, 330 ml, 320 ml, or 310 ml. When a disposable culture bag having a capacity of, for example, 21 is used, the lower limit of the volume per bag can be 100 ml, 200 ml, 300 ml, 400 ml, 500 ml, 600 ml, 700 ml, 800 ml, 900 ml, or 1000 ml, and the upper limit thereof can be 2000 ml, 1900 ml, 1800 ml, 1700 ml, 1600 ml, 1500 ml, 1400 ml, 1300 ml, 1200 ml, or 1100 ml. When a disposable culture bag having a capacity of 10 l is used, the lower limit of the volume per bag can be 500 ml, 1 l, 2 l, 3 l, 4 l, or 5 l, and the upper limit thereof can be 10 1, 9 1, 8 1, 7 1, or 61. When a reactor equipped with stirring blades of a capacity of interest is to be used, the volume of a medium or culture solution can be adjusted within a working volume designated by a manufacturer.

### (Seeding Density)

The density of the pluripotent stem cells after adherent culture to be seeded (seeding density) in a fresh medium in suspension culture is not particularly limited, and it can be appropriately adjusted in view of culture time, the condition of cells after culture, and the number of cells needed after culture. In general, the lower limit can be, for example, 0.01 × 10⁵ cells/ml, 0.1 × 10⁵ cells/ml, 1 × 10⁵ cells/ml, or 2 × 10⁵ cells/ml, and the upper limit can be, for example, 20 × 10⁵ cells/ml or 10 × 10⁵ cells/ml. It is particularly preferable that the lower limit of the seeding density be 2 × 10⁵ cells/ml and the upper limit thereof be 4 × 10⁵ cells/ml.

### (Culture Method)

The flowing state of a medium in suspension culture is not limited. Specifically, suspension culture may be performed by static culture or flow culture. Typically, suspension culture is performed by flow culture.

"Flow culture" refers to culture in which a medium is allowed to flow as described above. In the case of flow culture, a medium is preferably allowed to flow so as to promote cell aggregation. Examples of such culture methods include a rotational culture method, a rocking culture method, a stirring culture method, and combinations of any thereof.

The "rotational culture method" (including a shaking culture method) refers to a method of culture performed under conditions in which a medium is allowed to flow, so that cells can gather to one point by stress (centrifugal force or centripetal force) due to a rotational flow. Specifically, rotational culture is performed by rotating a culture vessel accommodating a medium containing cells along a closed trajectory, such as a circle, an ellipse, a flattened circle, or a flattened ellipse, on an approximately horizontal plane.

The rotational speed is not limited, and the lower limit can be, for example, 1 rpm, 10 rpm, 50 rpm, 60 rpm, 70 rpm, 80 rpm, 83 rpm, 85 rpm, or 90 rpm. The upper limit can be, for example, 200 rpm, 150 rpm, 120 rpm, 115 rpm, 110 rpm, 105 rpm, 100 rpm, 95 rpm, or 90 rpm. The amplitude of a shaker to be used for rotational culture is not particularly limited, and the lower limit can be, for example, 1 mm, 10 mm, or 20 mm. The upper limit can be, for example, 200 mm, 100 mm, 50 mm, or 30 mm. Likewise, the rotational radius in rotational culture is not particularly limited, and the rotational radius is preferably set to adjust the amplitude within the above range. The lower limit of the rotational radius can be, for example, 5 mm or 10 mm, and the upper limit thereof can be, for example, 100 mm or 50 mm. In the method for preparing a cell aggregate described below, in particular, rotation conditions adjusted within the above range are preferable because a cell aggregate of an adequate size can be readily prepared.

The "rocking culture method" refers to a method of culture performed under conditions in which a medium is provided with a rocking flow through linear reciprocating motion such as rocking stirring. Specifically, rocking culture is performed by rocking a culture vessel accommodating a medium containing cells on a plane approximately perpendicular to the horizontal plane. The rocking speed is not particularly limited, and rocking can be performed in such a manner that the lower limit as one reciprocating motion is regarded as one cycle is, for example, 2 cycles, 4 cycles, 6 cycles, 8 cycles, or 10 cycles per minute, and the upper limit is 50 cycles, 25 cycles, 20 cycles, or 15 cycles per minute. In rocking, it is preferable that the culture vessel be slightly inclined to the vertical plane; in other word, the culture vessel be provided with a rocking angle. The rocking angle is not particularly limited, and the lower limit can be, for example, 0.1°, 2°, 4°, 6°, or 8°, and the upper limit can be 20°, 18°, 15°, 12°, or 10°. In the method for preparing a cell aggregate described below, in particular, rocking conditions adjusted within the above range are preferable because a cell aggregate of an adequate size can be readily prepared.

Furthermore, culture can be performed with stirring by a motion composed of the rotation in combination with rocking.

The "stirring culture method" refers to a method of culture performed under conditions in which, while a culture vessel is allowed to stand, a medium in the culture vessel is stirred using a stirring device, such as a stirrer bar and a stirring blade. For example, stirring culture can be achieved using a spinner-flask-like culture vessel equipped with a stirring blade. Such culture vessels are commercially available, and they may be used. In the case of a commercially available spinner-flask-like culture vessel, a cell culture composition in an amount recommended by the manufacturer can be used in a favorable manner. The stirring speed of the stirring means is not particularly limited, and the lower limit can be 1 rpm, 10 rpm, 30 rpm, 50 rpm, 70 rpm, 90 rpm, 110 rpm, or 130 rpm. The upper limit can be 200 rpm or 150 rpm.

In the step of suspension culture, the number of cells attained by proliferation can be determined as desired. The target number of cells and the targeted cell conditions can be adequately determined in accordance with the types of cells to be cultured, the purpose of cell aggregation, the types of media, and culture conditions. In the case of suspension culture, for example, the number of cells (density) attained by proliferation can be 1 × 10⁶ cells/ml. While a size of each cell aggregate prepared is not particularly limited, the lower limit of the size in maximum width in an image observed under a microscope can be 30 µm, 40 µm, 50 µm, 60 µm, 70 µm, 80 µm, 90 µm, or 100 µm. The upper limit can be 1000 µm, 900 µm, 800 µm, 700 µm, 600 µm, 500 µm, 400 µm, 300 µm, or 200 µm. When the size of a cell aggregate is within such range, oxygen and nutrient components are easily supplied to the cells therein. Thus, a cell aggregate of such size is preferable as an environment for cell proliferation. It is particularly preferable that the lower limit of the size of a cell aggregate be 50 µm and the upper limit thereof be 300 µm.

It is preferable that 30% or more, 40% or more, 50% or more, 60% or more, 70% or more, 75% or more, 80% or more, 85% or more, 90% or more, 95% or more, 98% or more, or 100% of a population of cell aggregates to be prepared in the step of suspension culture be cell aggregates of the size range described above on a weight basis.

It is preferable that the percentage of viable cells (survival rate) in cells constituting a population of cell aggregates to be prepared by the step of suspension culture be, for example, 50% or higher, 60% or higher, 70% or higher, 80% or higher, or 90% or higher. Cell aggregates having a survival rate within the range can readily maintain the aggregated state and can thus provide preferable conditions for cell proliferation.

### 1-5-3. Step of Collection

In the "step of collection," the cultured pluripotent stem cells are collected from the culture solution after the step of adherent culture or the step of suspension culture. In the method for preparing a pluripotent stem cell population according to the present invention, the step of collection may or may not be performed. Specifically, the step of adherent culture may be followed by the step of collection, and the step of suspension culture may then be performed. Alternatively, the step of adherent culture may be performed, and the step of suspension culture may be performed immediately thereafter. Alternatively, the step of suspension culture may or may not be followed by the step of collection. The term "collection (of cells)" used herein refers to a procedure of separating cells from a culture solution to obtain cells. A method of collecting cells is not particularly limited, and a conventional method used in methods of cell culture in the art may be employed.

After the step of suspension culture, cells are present in a suspended state in the culture solution. Accordingly, collection of cells can be achieved by allowing the culture vessel to stand or performing centrifugation to remove a liquid component of the supernatant. Alternatively, cells may be collected using a filtration filter, a hollow fiber separation membrane, or the like. When a liquid component is removed by allowing to stand, the vessel containing the culture solution is left to stand for about 5 minutes, and the supernatant can be removed with precipitated cells or cell aggregates left unremoved. Centrifugation can be performed at a centrifugal acceleration for a period of a treatment time, so that cells are not damaged by the centrifugal force. The lower limit of the centrifugal acceleration is not particularly limited, as long as cells can be precipitated, and it can be, for example, 100 ×g, 300 ×g, 800 ×g, or 1000 ×g. The upper limit thereof can be adequately determined, so that cells are nod damaged or hardly damaged by the centrifugal force, and it can be, for example, 1600 ×g, 1500 ×g, or 1400 ×g. The lower limit of the treatment time is not particularly limited, provided that cells are precipitated by the centrifugal acceleration, and it can be, for example, 30 seconds, 1 minute, 3 minutes, or 5 minutes. The upper limit thereof can be adequately determined, so that cells are not damaged or hardly damaged by the centrifugal acceleration, and it can be, for example, 10 minutes, 8 minutes, 6 minutes, or 30 seconds. When a liquid component is to be removed by filtration, for example, the culture solution is passed through a nonwoven fabric or a mesh filter to remove the filtrate, and the residual cell aggregates can be collected. When a liquid component is to be removed with a hollow fiber separation membrane, for example, cells can be separated from the culture solution with use of an apparatus equipped with a hollow fiber separation membrane such as a Cell Washing Concentration System (KANEKA CORPORATION) and collected.

The cells collected can be washed, according to need. The washing method is not limited. For example, washing can be performed in the same manner as the washing method described in "Post-Treatment" in the step of adherent culture described above. As the washing solution, a buffer (including PBS buffer), physiological saline, or a medium (the basal medium is preferable) can be used.

### (Single-Cell Formation)

The pluripotent stem cells collected after the step of adherent culture or the step of suspension culture can be subjected to "single-cell formation." According to a process of "single-cell formation," a cell assembly in which a plurality of cells are adhered or aggregated with each other, such as a monolayer piece of cells or a cell aggregate, is dispersed to bring such assembly to the form of single, free cells. In the state of single, free cells, single cells separated from a monolayer piece of cells or a cell aggregate may be present. It is not necessary that all the cells constituting a monolayer piece of cells or a cell aggregate are in the form of single, free cells.

In single-cell formation, a detaching agent and/or a chelating agent is used. The detaching agent is not particularly limited, and, for example, trypsin, collagenase, Pronase, hyaluronidase, and elastase are applicable, and commercially available Accutase^{®}, Accumax^{®}, TrypLE^{™} Express Enzyme (Life Technologies Japan Ltd.), TrypLE^{™} Select Enzyme (Life Technologies Japan Ltd.), Dispase^{®}, and the like are also applicable. When trypsin is used for single-cell formation, for example, the lower limit of the concentration in the solution is not particularly limited, provided that a cell assembly can be dispersed, and the lower limit can be, for example, 0.15 vol%, 0.18 vol%, 0.20 vol%, or 0.24 vol%. The upper limit thereof is not particularly limited, provided that cells themselves are not dissolved or affected in other ways, and the upper limit can be, for example, 0.30 vol%, 0.28 vol%, or 0.25 vol%. While the treatment time depends on the concentration of trypsin, the lower limit is not particularly limited, provided that a cell assembly is sufficiently dispersed by the action of trypsin, and the lower limit can be, for example, 5 minutes, 8 minutes, 10 minutes, 12 minutes, or 15 minutes. The upper limit of the treatment time is not particularly limited, provided that cells themselves are not dissolved by the action of trypsin or affected in other ways, and the upper limit can be, for example, 30 minutes, 28 minutes, 25 minutes, 22 minutes, 20 minutes, or 18 minutes. When a commercially available detaching agent is used, the commercially available detaching agent can be used at a concentration that allows cells to be dispersed into single forms, as described in the attached protocol. Single-cell formation can be promoted by, for example, application of mild physical treatment on a monolayer piece of cells or a cell aggregate after the treatment with a detaching agent and/or a chelating agent. This physical treatment is not particularly limited, and examples thereof include multiple times of pipetting for cells together with a solution. In addition, cells may be passed through a strainer or a mesh, according to need.

The cells subjected to single-cell formation can be collected by removing the supernatant containing the detaching agent by allowing to stand or by centrifugation. The cells collected may be washed, according to need. The conditions for centrifugation and the washing method can be as described above.

### Examples

Hereafter, the method for producing a pluripotent stem cell population according to the present invention is described in greater detail with reference to the examples, although the technical scope of the present invention is not limited to the examples described below.

### (Comparative Example 1 (Comp. Ex. 1): Adherent culture of human iPS cells (1383D6))

Human iPS cells (1383D6) (Center for iPS Cell Research and Application, Kyoto University) were seeded at 10,000 cells/cm² on a cell culture dish coated with Vitronectin (VTN-N) Recombinant Human Protein, Truncated (Thermo Fisher Scientific) at 0.5 µg/cm², and subjected to adherent culture under at 37°C in the presence of 5% CO₂. The medium used was StemFit^{®} AK02N (Ajinomoto Co., Inc.), and medium exchange was performed every day. Only at the time of seeding the cells, Y-27632 (FUJIFILM Wako Pure Chemical Corporation) was added to the medium to the final concentration of 10 µM. The day of seeding of cells was defined as day 0 of culture, subculture was performed on day 4 of culture by treating the cells with Accutase (Innovative Cell Technologies, Inc.) for 5 minutes to detach the cells from the culture surface, and the cells were dispersed into single cells by pipetting. The cells were suspended in the StemFit^{®} AK02N medium containing Y-27632 at the final concentration of 10 µM, some of the cells were stained with trypan blue, and the number of viable cells was counted. Thereafter, the cells were seeded in the same manner, and adherent culture was continued.

### (Example 1 (Ex. 1)): Adherent culture of human iPS cells (1383D6))

Adherent culture was performed in the same manner as in Comparative Example 1, except that IWR-1-endo (20 µM) (FUJIFILM Wako Pure Chemical Corporation) and LY-333531 (1 µM) (Cayman Chemical Company) were added to the medium.

### (Example 2: Quantitative Real-Time PCR Analysis)

Quantitative real-time PCR analysis was performed with a procedure shown in the following. The cells on day 0 of culture in Comparative Example 1 and Example 1, the cells on day 8, day 16, and day 20 of culture in Comparative Example 1, and the cells on day 8, day 16, and day 20 of culture in Example 1 were dissolved using TRIzol^{™} Reagent (Thermo Fisher Scientific). From each solution resulting from dissolution with TRIzol^{™} Reagent, total RNA was isolated and purified using a PureLink^{®} RNA Mini kit (Thermo Fisher Scientific). The purified RNA was subjected to concentration measurement using a BioSpec-nano (Shimadzu Corporation), and RNA in an amount of 500 ng was separated. To the RNA separated, 2 µL of ReverTra Ace^{®} qPCR RT Master mix (TOYOBO CO., LTD.) and Rnase Free dH₂O were added to prepare 10 µL of a solution, and cDNA synthesis was performed using a SimpliAmp Thermal Cycler (Thermo Fisher Scientific). The reaction conditions for cDNA synthesis were such that reaction at 37°C for 15 minutes was followed by consecutive reactions of reaction at 50°C for 5 minutes and reaction at 98°C for 5 minutes, followed by cooling to 4°C. The synthesized cDNA solution was 100-fold diluted with 10 mM Tris-HCl pH 8.0 (NACALAI TESQUE, INC.), and added to a 384-well PCR plate (Thermo Fisher Scientific) at 5 µl/well. KOD SYBR^{®} qPCRMix (TOYOBO CO., LTD.), Forward primer prepared as 50 µM solution, Reverse primer prepared as 50 µM solution, and DEPC-treated water (NACALAI TESQUE, INC.) were mixed together at a ratio of 100:1:1:48, and this mixed solution was added to the 384-well PCR plate at 15 µl/well and mixed together. The primers used were those for ACTB, OCT4, SOX2, NANOG, TFCP2L1, OTX2, KLF4, PAX6, BRCHYURY, and SOX17. The 384-well PCR plate was centrifuged to remove bubbles in the wells, and quantitative real-time PCR analysis was carried out using a QuantStudio 7 Flex Real-Time PCR System (Thermo Fisher Scientific). Table 1 shows the reaction conditions.

**Table 1**

| Step | Temperature | Time | Number of cycles |
|---|---|---|---|
| 1 Initial denaturation | 98°C | 1 min | - |
| 2 Denaturation | 98°C | 15 sec | 5 cycles |
| 3 Annealing, elongation | 68°C | 30 sec | |
| 4 Denaturation | 98°C | 15 sec | 40 cycles |
| 5 Annealing | 60°C | 10 sec | |
| 6 Elongation | 68°C | 30 sec | |
| 7 Melting curve | 95°C | 15 sec | - |
| | 60°C | 1 min | |
| | 98°C | 15 sec | |

The nucleotide sequences of the primers used for the quantitative real-time PCR analysis are shown below.
ACTB (Forward): 5'-CCTCATGAAGATCCTCACCGA-3' (SEQ ID NO: 1)
ACTB (Reverse): 5'-TTGCCAATGGTGATGACCTGG-3' (SEQ ID NO: 2)
OCT4 (Forward): 5'-AGTGGGTGGAGGAAGCTGACAAC-3' (SEQ ID NO: 3)
OCT4 (Reverse): 5'-TCGTTGTGCATAGTCGCTGCTTGA-3' (SEQ ID NO: 4)
SOX2 (Forward): 5'-CACCAATCCCATCCACACTCAC-3' (SEQ ID NO: 5)
SOX2 (Reverse): 5'-GCAAAGCTCCTACCGTACCAC-3' (SEQ ID NO: 6)
NANOG (Forward): 5'-AGCCTCCAGCAGATGCAAGAACTC-3' (SEQ ID NO: 7)
NANOG (Reverse): 5'-TTGCTCCACATTGGAAGGTTCCCA-3' (SEQ ID NO: 8)

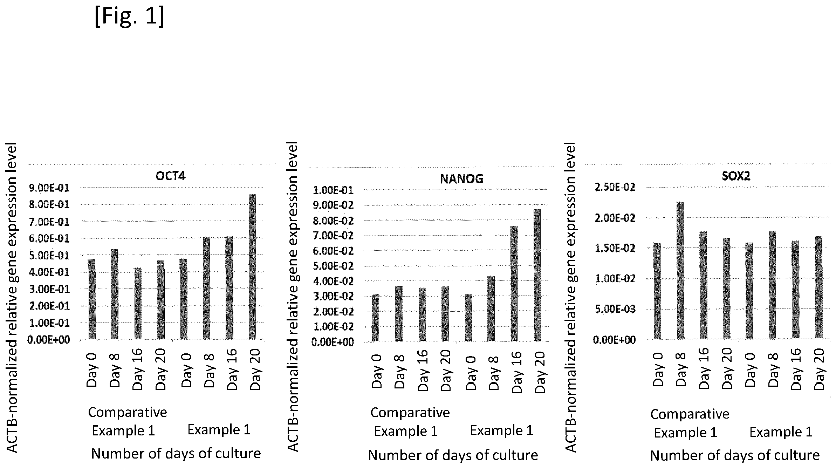

Table 2 and Fig. 1 show the results of measurement of gene expression.

**Table 2**

| ACTB-normalized relative gene expression level (2^{*-Δ*Ct}) | | | |
|---|---|---|---|
| | OCT4 | NANOG | SOX2 |
| Day 0 of Comp. Ex. 1 and Ex. 1 | 4.76 × 10⁻¹ | 3.10 × 10⁻² | 1.59 × 10⁻² |
| Day 8 of Comp. Ex. 1 | 5.34 × 10⁻¹ | 3.67 × 10⁻² | 2.26 × 10⁻² |
| Day 16 of Comp. Ex. 1 | 4.25 × 10⁻¹ | 3.57 × 10⁻² | 1.77 × 10⁻² |
| Day 20 of Comp. Ex. 1 | 4.69 × 10⁻¹ | 3.66 × 10⁻² | 1.66 × 10⁻² |
| Day 8 of Ex. 1 | 6.07 × 10⁻¹ | 4.31 × 10⁻² | 1.77 × 10⁻² |
| Day 16 of Ex. 1 | 6.08 × 10⁻¹ | 7.61 × 10⁻² | 1.60 × 10⁻² |
| Day 20 of Ex. 1 | 8.05 × 10⁻¹ | 8.68 × 10⁻² | 1.69 × 10⁻² |

As shown in Table 2 and Fig. 1, there was no significant difference in the expression levels of undifferentiation markers when adherent culture was performed with the use of a culture solution containing a PKCβ inhibitor and a TNKS inhibitor, compared with the cells cultured with the use of a culture solution not containing a PKCβ inhibitor or a TNKS inhibitor while maintaining the undifferentiated state (Comparative Example 1). The results demonstrate that pluripotent stem cells remain undifferentiated even when adherent culture was performed with the use of a culture solution containing a PKCβ inhibitor and a TNKS inhibitor.

### (Example 3: Flow Cytometry Analysis)

The cells subjected to adherent culture in the same manner as in Comparative Example 1 and Example 1 were treated with Accutase on day 20 of culture and dispersed into single cells by pipetting. The cells were washed with PBS (phosphate-buffered saline). Thereafter, the cells were fixed, permeabilized, and blocked using the eBioscience Foxp3 Transcription factor staining buffer set (Thermo Fisher Scientific). Thereafter, each cell sample was divided into 4 and resuspended in 50 µl each buffer included in the eBioscience Foxp3 Transcription factor staining buffer set (Thermo Fisher Scientific). To one of the cell suspensions, fluorescence-labeled anti-OCT4, anti-SOX2, and anti-NANOG antibodies were added and mixed. To each of other three cell suspensions, 2 of the aforementioned 3 fluorescence-labeled antibodies were added and mixed to prepare FMO control samples. Staining was performed at 4°C under shading for 1 hour. Table 3 shows the antibodies used and the amounts thereof added.

**Table 3**

| | Model number, manufacturer | Amount added |
|---|---|---|
| Fluorescence-labeled anti-OCT4 antibody | 5601786, Becton, Dickinson and Company | 10 µl |
| Fluorescence-labeled anti-SOX2 antibody | 656110, Becton, Dickinson and Company | 1 µl |
| Fluorescence-labeled anti-NANOG antibody | 561300, Becton, Dickinson and Company | 2.5 µl |

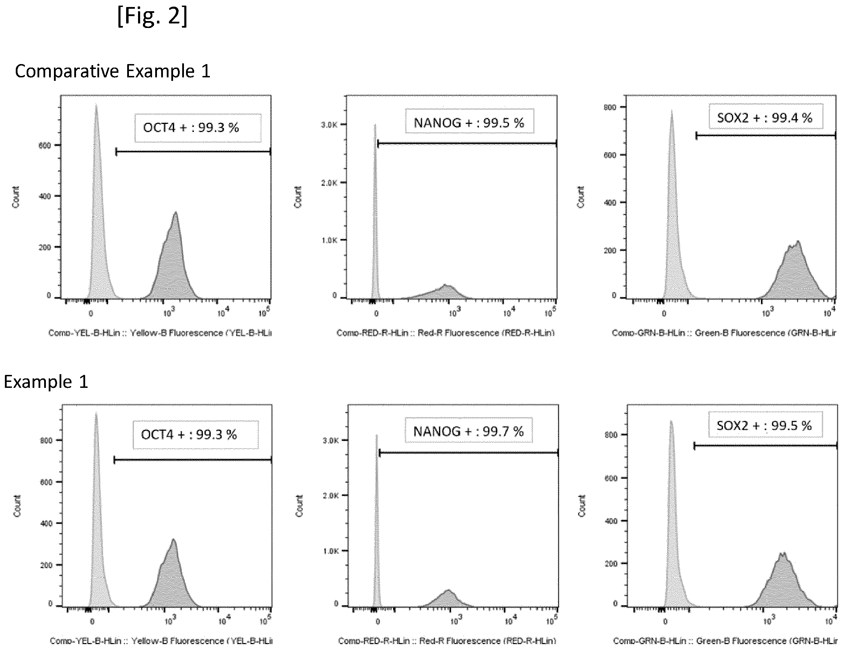

After washing once with 3% FBS (fetal bovine serum)/PBS, the cells passed through a cell strainer were analyzed with a Guava easyCyte 8HT (Luminex Corporation). For FMO control samples, all regions where the proportion of cell populations with higher fluorescence intensity among cell populations extracted by the FSC/SSC dot plots was 1.0% or lower were selected. For the sample treated with the anti-OCT4, anti-SOX2, and anti-NANOG antibodies, the proportion of cells included in the regions in the cell populations extracted by the FSC/SSC dot plots were calculated, and the resultants were used as the proportions of cells positive for OCT4, SOX2, and NANOG. The results are shown in Table 4 and Fig. 2.

**Table 4**

| | Proportion (%) of cells positive for OCT4 | Proportion (%) of cells positive for SOX2 | Proportion (%) of cells positive for NANOG |
|---|---|---|---|
| Comp. Ex. 1 | 99.3 | 99.4 | 99.4 |
| Ex. 1 | 99.3 | 99.5 | 99.7 |

As shown in Table 4 and Fig. 2, the proportions of cells positive for the undifferentiation markers OCT4, SOX2, and NANOG were 90% or higher when adherent culture was performed with the use of a culture solution containing a PKCβ inhibitor and a TNKS inhibitor, as with the case of the cells cultured with the use of a conventional culture solution not containing a PKCβ inhibitor or a TNKS inhibitor while maintaining the undifferentiated state (Comparative Example 1). The results demonstrate that, even when adherent culture is performed with the addition of the PKCβ inhibitor and the TNKS inhibitor, a cell population deviated from the undifferentiated state would not appear, and a pluripotent stem cell population maintaining the undifferentiated state can be obtained.

### (Comparative Example 2: Transition from adherent culture of human iPS cells (1383D6) to suspension culture thereof)

The human iPS cells (1383D6) subjected to adherent culture in the same manner as in Comparative Example 1 were treated with Accutase (Innovative Cell Technologies, Inc.) for 5 minutes on day 12 of culture to detach the cells from the culture surface, and the cells were dispersed into single cells by pipetting. The cells were suspended in the StemFit^{®} AK02N medium containing Y-27632 at the final concentration of 10 µM, some of the cells were stained with trypan blue, and the number of viable cells was counted. A cell suspension was prepared to contain 2 × 10⁵ cells per 1 ml with the use of the StemFit^{®} AK02N medium containing Y-27632 at the final concentration of 10 µM. The cell suspension was seeded on a 6-well plate for suspension culture (Sumitomo Bakelite Co., Ltd.) at 4 ml/well. The plate comprising the cells seeded thereon was allowed to rotate to form a circle with a rotation width (diameter) of 25 mm on the horizontal plane at 90 rpm on a rotary shaker, and suspension culture was performed at 37°C in the presence of 5% CO₂.

### (Example 4: Transition from adherent culture of human iPS cells (1383D6) to suspension culture thereof)

The human iPS cells (1383D6) subjected to adherent culture in the same manner as in Example 1 were subjected to suspension culture on day 12 of culture in the same manner as in Comparative Example 2.

### (Example 5: Measurement of cell count attained by suspension culture of human iPS cells (1383D6))

On the day following the day on which a method of cell culture was switched from adherent culture to suspension culture in the manner described in Comparative Example 2 and Example 4, cell aggregates and a culture supernatant were collected from wells to a centrifuge tube, the centrifuge tube was allowed to stand for approximately 5 minutes to precipitate the cell aggregates, and the culture supernatant was removed. Accutase (1 ml) was added to the cell aggregates, the cell aggregates were treated at 37°C for 10 minutes, and the cells were dispersed into single cells by pipetting. The cells were suspended in the StemFit^{®} AK02N medium containing Y-27632 at the final concentration of 10 µM, some of the cells were stained with trypan blue, and the number of viable cells was counted. The measured cell counts are shown in Table 5.

**Table 5**

| Cell count per 1 ml of culture solution (cells/ml) | |
|---|---|
| Comparative Example 1 | Example 1 |
| 1.85 × 10⁵ | 3.30 × 10⁵ |

As shown in Table 5, the cell count measured in Example 4 was larger than that in Comparative Example 2. This indicates that the cell death caused upon transition from adherent culture to suspension culture can be prevented and efficiency is improved to a significant extent according to the present invention.

### (Comparative Example 3: Suspension culture of human iPS cells (1383D6))

The human iPS cells (1383D6) subjected to adherent culture in the same manner as in Comparative Example 1 were treated with Accutase (Innovative Cell Technologies, Inc.) for 5 minutes on day 4 of culture to detach the cells from the culture surface, and the cells were dispersed into single cells by pipetting. The cells were suspended in the StemFit^{®} AK02N medium containing Y-27632 at the final concentration of 10 µM, some of the cells were stained with trypan blue, and the number of viable cells was counted. A cell suspension was prepared to contain 2 × 10⁵ cells per 1 ml with the use of the StemFit^{®} AK02N medium containing Y-27632 at the final concentration of 10 µM. The cell suspension was seeded on a 6-well plate for suspension culture (Sumitomo Bakelite Co., Ltd.) at 4 ml/well. The plate comprising the cells seeded thereon was allowed to rotate to form a circle with a rotation width (diameter) of 25 mm on the horizontal plane at 90 rpm on a rotary shaker, and suspension culture was performed at 37°C in the presence of 5% CO₂.

### (Example 6: Suspension culture of human iPS cells (1383D6))

The human iPS cells (1383D6) subjected to adherent culture in the same manner as in Example 1 were subjected to suspension culture on day 4 of culture in the same manner as in Comparative Example 2.

### (Example 7: Measurement of cell count attained by suspension culture of human iPS cells (1383D6))

On the day following the day on which a method of cell culture was switched from adherent culture to suspension culture in the manner described in Comparative Example 3 and Example 6, cell aggregates and a culture supernatant were collected from wells to a centrifuge tube, the centrifuge tube was allowed to stand for approximately 5 minutes to precipitate the cell aggregates, and the culture supernatant was removed. Accutase (1 ml) was added to the cell aggregates, the cell aggregates were treated at 37°C for 10 minutes, and the cells were dispersed into single cells by pipetting. The cells were suspended in the StemFit^{®} AK02N medium containing Y-27632 at the final concentration of 10 µM, some of the cells were stained with trypan blue, and the number of viable cells was counted. The measured cell counts are shown in Table 6.

**Table 6**

| Cell count per 1 ml of culture solution (cells/ml) | |
|---|---|
| Comparative Example 1 | Example 1 |
| 1.54 × 10⁵ | 2.01 × 10⁵ |

As shown in Table 6, the cell count measured in Example 6 was larger than that in Comparative Example 3. This indicates that the cell death caused upon transition from adherent culture to suspension culture can be prevented and efficiency is improved to a significant extent according to the present invention.

### (Example 8: Adherent culture of human iPS cells (1383D6))

Human iPS cells (1383D6) (Center for iPS Cell Research and Application, Kyoto University) were seeded at 1,000 cells/cm² in a cell culture flask coated with iMatrix-511 (Matrixome Inc.) at 0.5 µg/cm² and subjected to adherent culture at 37°C in the presence of 5% CO₂. The medium used was StemFit^{®} AK02N (Ajinomoto Co., Inc.). The day of seeding of cells was defined as day 0 of culture, and medium exchange was performed on day 1, day 4, day 6, and day 7 of culture. Only at the time of seeding the cells, Y-27632 (FUJIFILM Wako Pure Chemical Corporation) was added to the medium to the final concentration of 10 µM. For medium exchange performed on day 4 of culture and thereafter, StemFit^{®} AK02N (Ajinomoto Co., Inc.) supplemented with IWR-1-endo (20 µM) (FUJIFILM Wako Pure Chemical Corporation) and LY-333531 (1 µM) (Cayman Chemical Company) was used. On day 8 of culture, the cells were treated with TrypLE SELECT (Thermo Fisher Scientific) for subculture, the cells were detached from the culture surface, and the cells were dispersed into single cells by pipetting. The cells were suspended in the StemFit^{®} AK02N medium containing Y-27632 at the final concentration of 10 µM and IWR-1-endo (20 µM) (FUJIFILM Wako Pure Chemical Corporation), some of the cells were stained with trypan blue, and the number of viable cells was counted. Thereafter, the cells were seeded in the BioBLU 1c reactor (Eppendorf) using the StemFit^{®} AK02N medium containing Y-27632 at the final concentration of 10 µM and IWR-1-endo (FUJIFILM Wako Pure Chemical Corporation) at the final concentration of 20 µM to bring the liquid amount to 320 ml and the cell density to 100,000 cells/ml. At 37°C in the presence of 5% CO₂, stirred suspension culture was initiated 75 rpm.

### (Example 9: Measurement of cell count attained by suspension culture of human iPS cells (1383D6))

On the day following the day on which a method of cell culture was switched from adherent culture to suspension culture in the manner described in Example 8, 3 ml of a culture solution was collected from the reactor to a centrifuge tube, the centrifuge tube was allowed to stand for approximately 5 minutes to precipitate the cell aggregates, and the culture supernatant was removed. Accutase (1 ml) was added to the cell aggregates, the cell aggregates were treated at 37°C for 10 minutes, and the cells were dispersed into single cells by pipetting. The cells were suspended in the StemFit^{®} AK02N medium containing Y-27632 at the final concentration of 10 µM, some of the cells were stained with trypan blue, and the number of viable cells was counted. The measured cell counts are shown in Table 7.

**Table 7**

| Cell count per 1 ml of culture solution (cells/ml) |
|---|
| 148,000 |

As shown in Table 7, the cell count on the day following the day on which the cells were subjected to suspension culture was significantly large in Example 8, and the number of cells was larger than the number of the cells seeded. In general, the cell count on the day following the day on which the cells were subjected to suspension culture becomes smaller than the number of the cells seeded because of damages caused by enzyme treatment at the time of subculture, cell death in the form of single cells without forming aggregates. According to the method of the present invention, however, such problems can be suppressed and productivity can be improved to a significant extent not only by rotated suspension culture but also by stirred suspension culture.

## Claims

1. A method for producing a pluripotent stem cell population comprising:
a step of adherent culture of pluripotent stem cells in a liquid medium comprising a PKCβ inhibitor and a TNKS inhibitor; and
a step of suspension culture of the pluripotent stem cells after adherent culture.

2. The method according to Claim 1, wherein concentration of the PKCβ inhibitor in the liquid medium is 25 nM to 15 µM.

3. The method according to Claim 1, wherein concentration of the TNKS inhibitor in the liquid medium is 90 nM to 40 µM.

4. The method according to Claim 1, wherein the liquid medium comprises at least one substance selected from the group consisting of L-ascorbic acid, insulin, transferrin, selenium, and sodium bicarbonate.

5. The method according to Claim 1, wherein the liquid medium comprises FGF2 and/or TGF-β1.

6. The method according to Claim 1, wherein the liquid medium comprises a ROCK inhibitor.

7. The method according to Claim 6, wherein the ROCK inhibitor is Y-27632.

8. The method according to Claim 1, wherein the step of suspension culture comprises a step of forming a cell aggregate.

9. The method according to Claim 1, wherein the step of suspension culture comprises a step of collecting a cell aggregate.

10. The method according to Claim 1, wherein, in the pluripotent stem cell population, a proportion of cells positive for OCT4 is 90% or higher, that of cells positive for SOX2 is 90% or higher, and that of cells positive for NANOG is 90% or higher.

11. The method according to Claim 1, wherein the pluripotent stem cells are ES cells and/or induced pluripotent stem cells.

12. The method according to Claim 1, wherein, in the step of suspension culture of pluripotent stem cells, the pluripotent stem cells after adherent culture are sowed in a liquid medium not comprising PKCβ inhibitor and/or TNKS inhibitor.
